(19) Europäisches Patentamt
European Patent Office
Office européen des brevets

(11) **EP 2 560 550 B1**

(12) **EUROPEAN PATENT SPECIFICATION**

(45) Date of publication and mention
of the grant of the patent:
**06.12.2017 Bulletin 2017/49**

(51) Int Cl.:
***A61B 5/08*** (2006.01)

(21) Application number: **11772576.2**

(86) International application number:
**PCT/US2011/033100**

(22) Date of filing: **19.04.2011**

(87) International publication number:
**WO 2011/133582 (27.10.2011 Gazette 2011/43)**

(54) **BODY-WORN MONITOR FOR MEASURING RESPIRATORY RATE**

AM KÖRPER GETRAGENER MONITOR ZUR MESSUNG DER ATEMFREQUENZ

DISPOSITIF DE SURVEILLANCE PORTÉ SUR LE CORPS POUR LA MESURE DE FRÉQUENCES RESPIRATOIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.04.2010 US 762963
19.04.2010 US 762952
19.04.2010 US 762944
19.04.2010 US 762936
19.04.2010 US 762925
19.04.2010 US 762874**

(43) Date of publication of application:
**27.02.2013 Bulletin 2013/09**

(73) Proprietor: **Sotera Wireless, Inc.
San Diego, CA 92121 (US)**

(72) Inventors:
• **BANET, Matt**
**Kihei, HI 96753 (US)**
• **DHILLON, Marshal**
**San Diego, CA 92127 (US)**
• **McCOMBIE, Devin**
**San Diego, CA 92130 (US)**

(74) Representative: **Schiweck, Weinzierl & Koch
Patentanwälte Partnerschaft mbB
Landsberger Straße 98
80339 München (DE)**

(56) References cited:
EP-A1- 0 875 199     US-A- 3 608 542
US-A- 6 081 742     US-A1- 2005 240 087
US-A1- 2008 221 399     US-A1- 2009 118 626
US-A1- 2010 030 034     US-A1- 2010 056 881
US-B1- 6 236 872

**Description**

BACKGROUND OF THE INVENTION

Field of the Invention

The present invention relates to medical devices for monitoring vital signs, e.g., respiratory rate (RR).

Description of the Related Art

RR is a vital sign typically measured in hospitals using either an indirect, electrode-based technique called 'impedance pneumography' (IP), a direct optical technique called 'end-tidal CO2' (et-CO2), or simply through manual counting of breaths by a medical professional. IP is typically performed in lower-acuity areas of the hospital, and uses the same electrodes which measure an electrocardiogram (ECG) and corresponding heart rate (HR). These electrodes are typically deployed in a conventional 'Einthoven's triangle' configuration on the patient's torso. During IP, one of the electrodes supplies a low-amperage ($\sim$4 mA) current that is typically modulated at a high frequency ($\sim$50-100 kHz). Current passes through the patient's thoracic cavity, which is characterized by a variable, time-dependent capacitance that varies with each breath. A second electrode detects current which is modulated by the changing capacitance. Ultimately this yields an analog signal that is processed with a series of amplifiers and filters to detect the time-dependent capacitance change and, with subsequent analysis, the patient's RR.

In et-CO2, a device called a capnometer features a small plastic tube that inserts in the patient's mouth. With each breath the tube collects expelled CO2. A beam of infrared radiation emitted from an integrated light source passes through the CO2 and is absorbed in a time-dependent manner that varies with the breathing rate. A photodetector and series of processing electronics analyze the transmitted signal to determine RR. et-CO2 systems are typically used in high-acuity areas of the hospital, such as the intensive care unit (ICU), where patients often need ventilators to assist them in breathing.

In yet another technique, RR can be measured from the envelope of a time-dependent optical waveform called a photoplethysmogram (PPG) that is measured from the patient's index finger during a conventional measurement of the patient's oxygen saturation (SpO2). Breathing changes the oxygen content in the patient's blood and, subsequently, its optical absorption properties. Such changes cause a slight, low-frequency variation in the PPG that can be detected with a pulse oximeter's optical system, which typically operates at both red and infrared wavelengths.

Not surprisingly, RR is an important predictor of a decompensating patient. For example, a study in 1993 concluded that a RR greater than 27 breaths/minute was the most important predictor of cardiac arrests in hospital wards (Fieselmann et al., 'RR predicts cardiopulmonary arrest for internal medicine patients', J Gen Intern Med 1993; 8: 354-360). Subbe et al. found that, in unstable patients, relative changes in RR were much greater than changes in heart rate or systolic blood pressure; RR was therefore likely to be a better means of discriminating between stable patients and patients at risk (Subbe et al., 'Effect of introducing the Modified Early Warning score on clinical outcomes, cardio-pulmonary arrests and intensive care utilization in acute medical admissions', Anaesthesia 2003; 58: 797-802). Goldhill et al. reported that 21% of ward patients with a RR of 25-29 breaths/minute assessed by a critical care outreach service died in hospital (Goldhill et al., 'A physiologically-based early warning score for ward patients: the association between score and outcome', Anaesthesia 2005; 60: 547-553). Those with a higher RR had even higher mortality rates. In another study, just over half of all patients suffering a serious adverse event on the general wards (e.g. a cardiac arrest or ICU admission) had a RR greater than 24 breaths/minute. These patients could have been identified as high risk up to 24 hours before the event with a specificity of over 95% (Cretikos et al., 'The Objective Medical Emergency Team Activation Criteria: a case-control study', Resuscitation 2007; 73: 62-72). Medical references such as these clearly indicate that an accurate, easy-to-use device for measuring RR is an important component for patient monitoring within the hospital.

Despite its importance and the large number of available monitoring techniques, RR is notoriously difficult to measure, particularly when a patient is moving. During periods of motion, non-invasive techniques based on IP and PPG signals are usually overwhelmed by artifacts, and thus completely ineffective. This makes it difficult or impossible to measure RR from an ambulatory patient. Measurements based on et-CO2 are typically less susceptible to motion, but require a plastic tube inserted in the patient's mouth, which is uncomfortable and typically impractical for ambulatory patients. Document EP0875199 discloses a system for measuring respiratory rate from a patient, comprising: an optical pulse wave sensor; a motion sensor connected to the patient and configured to measure at least one motion signal corresponding to motion of a portion of the patient's body to which it is attached; and a processing system configured to receive the impedance pneumography signal and process it to determine a frequency-domain impedance pneumography spectrum, and to receive the motion signal and process it to determine a frequency-domain motion spectrum, the processing system further configured to collectively process both the impedance pneumography spectrum and motion spectrum to remove motion components from the impedance pneumography spectrum and then determine respiratory rate.

## SUMMARY OF THE INVENTION

This invention provides a technique for measuring RR using multiple input signals, including IP and accelerometer waveforms (ACC), and is defined in claims 1 and 6. After being measured with a body-worn system, an algorithm collectively analyzes these waveforms to determine RR from an ambulatory patient using combinations of simple peak counting, Fourier Transforms (FFT) and adaptive filtering. The patient's degree of motion determines which of these algorithms is implemented: simple peak counting is preferably used when the patient is undergoing no motion, while the FFT-based algorithm is used when motion is extreme. Adaptive filtering is typically used during periods of moderate motion. The algorithms are typically performed using a microprocessor, computer code and memory located in a wrist-worn transceiver, a sensor module located directly on the patient's chest, or on a remote server located, e.g., in a hospital. Calculations may be performed in a distributed manner, meaning portions of them can be performed with a first microprocessor (e.g., the server in the hospital), resulting in parameters that are then sent to a second microprocessor (e.g., in the wrist-worn transceiver) for final processing. Such a distributed model can reduce the computational burden on microprocessors within the body-worn monitor, thereby conserving power and extending battery life.

The accelerometer is typically mounted on the patient's torso (most typically the chest or belly), and measures small, breathing-induced movements to generate the time-dependent ACC waveform. The ACC waveform is also highly sensitive to the patient's motion and position, and thus the ACC waveform can be processed to determine parameters such as degree of motion, posture, and activity level. With the FFT-based algorithms, time-domain ACC and IP waveforms are mathematically transformed to the frequency domain and processed to generate a power spectrum. Further processing of this signal yields frequency components corresponding to both respiratory events and motion. The ACC waveform yields well-defined frequency components that are highly sensitive to motion. These signals can be collectively processed and used to filter out motion artifacts from the transformed IP waveform. The resulting power spectrum is then further processed with a smoothing function, yielding a set of frequency-domain peaks from which RR can be accurately calculated.

The multi-component algorithm also processes both IP and ACC waveforms to determine parameters for an adaptive filtering calculation. Once the parameters are determined, this filter is typically implemented with a finite impulse response (FIR) function. Ultimately this yields a customized filtering function which then processes the IP waveform to generate a relatively noise-free waveform with well-defined pulses corresponding to RR. Each pulse can then be further processed and counted to determine an accurate RR value, even during periods of motion.

The body-worn monitor measures IP and ACC waveforms as described above, along with PPG and ECG waveforms, using a series of sensors integrated into a comfortable, low-profile system that communicates wirelessly with a remote computer in the hospital. The body-worn monitor typically features three accelerometers, each configured to measure a unique signal along its x, y, and z axes, to yield a total of nine ACC waveforms. Typically the accelerometers are embedded in the monitor's cabling or processing unit, and are deployed on the patient's torso, upper arm, and lower arm. Each ACC waveform can be additionally processed to determine the patient's posture, degree of motion, and activity level. These parameters serve as valuable information that can ultimately reduce occurrences of 'false positive' alarms/alerts in the hospital. For example, if processing of additional ACC waveforms indicates a patient is walking, then their RR rate, which may be affected by walking-induced artifacts, can be ignored by an alarm/alert engine associated with the body-worn monitor. The assumption in this case is that a walking patient is likely relatively healthy, regardless of their RR value. Perhaps more importantly, with a conventional monitoring device a walking patient may yield a noisy IP signal that is then processed to determine an artificially high RR, which then triggers a false alarm. Such a situation can be avoided with an independent measurement of motion, such as that described herein. Other heuristic rules based on analysis of ACC waveforms may also be deployed according to this invention.

Sensors attached to the wrist and bicep each measure signals that are collectively analyzed to estimate the patient's arm height; this can be used to improve accuracy of a continuous blood pressure measurement (cNIBP), as described below, that measures systolic (SYS), diastolic (DIA), and mean (MAP) arterial blood pressures. And the sensor attached to the patient's chest measures signals that are analyzed to determine posture and activity level, which can affect measurements for RR, SpO2, cNIBP, and other vital signs. Algorithms for processing information from the accelerometers for these purposes are described in detail in the following patent applications, BODY-WORN MONITOR FEATURING ALARM SYSTEM THAT PROCESSES A PATIENT'S MOTION AND VITAL SIGNS (U.S.S.N 12/469,182; filed May 20, 2009) and BODY-WORN VITAL SIGN MONITOR WITH SYSTEM FOR DETECTING AND ANALYZING MOTION (U.S.S.N 12/469,094; filed May 20, 2009). As described therein, knowledge of a patient's motion, activity level, and posture can greatly enhance the accuracy of alarms/alerts generated by the body-worn monitor.

The body-worn monitor features systems for continuously monitoring patients in a hospital environment, and as the patient transfers from different areas in the hospital, and ultimately to the home. Both SpO2 and cNIBP rely on accurate measurement of PPG and ACC waveforms, along with an ECG, from patients that are both moving and at rest.

cNIBP is typically measured with the 'Composite Technique', which is described in detail in the co-pending patent applications entitled: VITAL SIGN MONITOR FOR MEASURING BLOOD PRESSURE USING OPTICAL, ELECTRICAL, AND PRESSURE WAVEFORMS (US 8419649 B2) and BODY-WORN SYSTEM FOR MEASURING CONTINUOUS, NON-INVASIVE BLOOD PRESSURE (CNIBP) (US 8808188 B2).

As described in these applications, the Composite Technique (or, alternatively, the 'Hybrid Technique', as referred to therein) typically uses a single PPG waveform from the SpO2 measurement (typically generated with infrared radiation), along with the ECG waveform, to calculate a parameter called 'pulse transit time' (PTT) which strongly correlates to blood pressure. Specifically, the ECG waveform features a sharply peaked QRS complex that indicates depolarization of the heart's left ventricle, and, informally, provides a time-dependent marker of a heart beat. PTT is the time separating the peak of the QRS complex and the onset, or 'foot', of the PPG waveforms. The QRS complex, along with the foot of each pulse in the PPG, can be used to more accurately extract AC signals using a mathematical technique described in detail below. In other embodiments both the red and infrared PPG waveforms are collectively processed to enhance the accuracy of the cNIBP measurement.

The electrical system for measuring IP and ACC waveforms is featured in a sensor module that connects to an end of a cable that terminates in the wrist-worn transceiver, and is mounted directly on the patient's chest. The sensor module measures high-fidelity digital waveforms which pass through the cable to a small-scale, low-power circuit mounted on a circuit board that fits within the transceiver. There, an algorithm processes the two waveforms using the multi-component algorithm to determine RR. The transceiver additionally includes a touchpanel display, barcode reader, and wireless systems for ancillary applications described, for example, in the above-referenced applications.

In one aspect, the invention features a system for measuring RR from a patient. The system includes an IP sensor, connected to at least two electrodes, and a processing system that receives and processes signals from the electrodes to measure an IP signal. The electrodes can connect to the IP sensor through either wired or wireless means. A motion sensor (e.g. an accelerometer) measures at least one motion signal (e.g. an ACC waveform) describing movement of a portion of the patient's body to which it is attached. The processing system receives the IP and motion signals, and processes them to determine, respectfully, frequency-domain IP and motion spectra. Both spectra are then collectively processed to remove motion components from the IP spectrum and determine RR. For example, during the processing, an algorithm determines motion frequency components from the frequency-domain motion spectrum, and then using a digital filter removes these, or parameters calculated therefrom, from the IP spectrum.

In embodiments, a single sensor module, adapted to be worn on the patient's torso, encloses both the IP sensor and the motion sensor. The sensor module typically includes at least one analog-to-digital converter configured to digitize the IP signal; this component may be integrated directly into a single-chip circuit (e.g. an application-specific integrated circuit, or ASIC), or in a circuit consisting of a collection of discrete components (e.g. individual resistors and capacitors). The sensor module can also include at least one analog-to-digital converter configured to digitize the motion signal. Similarly, this component can be integrated directly into the accelerometer circuitry. Digitizing the IP and motion signals before transmitting them to the processing system has several advantages, as described in detail below.

In other embodiments, the sensor module includes a temperature sensor for measuring the patient's skin temperature, and an ECG circuit (corresponding to a three, five, or twelve-lead ECG) for measuring an ECG waveform. In embodiments, the sensor module simply rests on the patient's chest during a measurement, or can be connected with a small piece of medical tape. Alternatively, the housing features a connector that connects directly to an ECG electrode worn on the patient's torso.

The processing system is typically worn on the patient's wrist. Alternatively, this system can be within the sensor module, or within a remote computer server (located, e.g., in a hospital's IT system). Typically a wireless transceiver (e.g. a transceiver based on 802.11 or 802.15.4 transmission protocols) is included in the system, typically within the processing module. Such a transceiver, for example, can wirelessly transmit IP and ACC waveforms to a remote processing system for further analysis. In this case, the processing system is further configured to wireless transmit a RR value back to a second processor worn on the patient's body, where it can then be displayed (using, e.g., a conventional display).

Accelerometers used within the system typically generate a unique ACC waveform corresponding to each axis of a coordinate system. In embodiments the system can include three accelerometers, each worn on a different portion of the patient's body. Waveforms generated by the accelerometers can then be processed as described in detail below to determine the patient's posture.

In another aspect, the invention features an algorithm, typically implemented using compiled computer code, a computer memory, and a microprocessor, that processes IP and ACC waveforms by calculating their power spectra by way of a Fourier transform (e.g. a fast Fourier transform, of FFT). The algorithm then determines motion components from the frequency-dependent ACC spectrum, and using a digital filter removes these, or components calculated therefrom, from the frequency-dependent IP spectrum. This yields a processed, frequency-dependent IP spectrum which can then be analyzed as described in detail below to estimate RR, even when large amounts of motion-induced noise are evident on the IP signal.

In embodiments, power spectra for both the IP and ACC waveforms are calculated from a complex FFT that includes both real and imaginary components. In other embodiments, alternative mathematical transforms, such as a Laplace transform, can be used in place of the FFT.

To determine a digital filter from the ACC power spectrum, the algorithm typically includes a method for first finding a peak corresponding to one or more frequencies related to the patient's motion. A bandpass filter, characterized by a passband which filters out these (and related) frequencies, is then generated and used to process the IP spectrum. Alternatively, these frequencies can simply be divided or subtracted from the IP spectrum. In all cases, this yields a processed IP spectrum which is then further analyzed to determine a frequency corresponding to RR. Analysis can include smoothing, averaging, or related methodologies uses to extract a single frequency, corresponding to RR, from a collection of frequencies. In embodiments, the algorithm can also include a component that generates an alarm if the patient's RR is greater than a first pre-determined threshold, or less than a second pre-determined threshold. The alarm can be generated by considering both the patient's RR and posture.

In another aspect, the invention features a multi-component algorithm for determining RR. The multi-component algorithm first determines a motion parameter from the ACC waveform. The motion parameter indicates the patient's degree of motion, activity level, or posture. Based on the motion parameter, the multi-component algorithm then selects one of the following algorithms to process one or both of the ACC and IP waveforms to determine RR: i) a first algorithm featuring counting breathing-induced pulses in the IP waveform; and ii) a second algorithm featuring collectively processing both the ACC and IP waveform to determine a digital adaptive filter, and then processing one of these waveforms with the adaptive filter to determine RR; and iii) a third algorithm featuring mathematically transforming both the ACC and IP waveforms into frequency-domain spectra, and then collectively processing the spectra to determine RR. Typically the first, second, and third algorithms are deployed, respectively, when the motion parameter indicates the patient's motion is non-existent, minimal, or large. For example, the first algorithm is typically deployed when the patient is resting; the second algorithm deployed when the patient is moving about somewhat; and the third algorithm deployed when the patient is standing up, and possibly walking or even running.

In another aspect, the multi-component algorithm is deployed on one or more microprocessors associated with the system. For example, to conserve battery life of the body-worn monitor, numerically intensive calculations (such as the FFT or those used to generate the digital filter) can be performed on a remote server; intermediate or final parameters associated with these calculations can then be wirelessly transmitted back to the body-worn monitor for further processing or display. In another embodiment, portions of the multi-component algorithm can be carried out by microprocessors located in both the wrist-worn transceiver and chest-worn sensor module. The microprocessors can communicate through serial or wireless interfaces. This latter approach will have little impact on battery life, but can reduce processing time by simultaneously performing different portions of the calculation.

In another aspect, the invention provides a method for measuring RR from a patient using an algorithm based on a digital adaptive filter. In this approach, the body-worn monitor measures both IP and ACC waveforms as described above. The waveforms are then collectively processed to determine a set of coefficients associated with the adaptive filter. Once calculated, the coefficients are stored in a computer memory. At a later point in time, the monitor measures a second set of IP and ACC waveforms, and analyzes these to determine a motion parameter. When the motion parameter exceeds a pre-determined threshold, the algorithm processes the set of coefficients and the latest IP waveform to determine a processed IP waveform, and then analyzes this to determine RR.

In embodiments, the digital adaptive filter is calculated from an impulse response function, which in turn is calculated from either a FIR function or an autoregressive moving average model. The order of the adaptive filter calculated from the impulse response function is typically between 20 and 30, while the order of the filter calculated from the autoregressive moving average model is typically between 1 and 5. A specific mathematic approach for calculating the digital adaptive filter is described below with reference to Eqs. 1-16. As described above, the coefficients can be calculated using a microprocessor located on the wrist-worn transceiver, sensor module, or a remote server.

In yet another aspect, the invention provides a system for measuring RR featuring a sensor module configured to be worn on the patient's torso. The sensor module includes sensors for measuring both IP and ACC waveforms, and a serial transceiver configured to transmit the digital signals through a cable to a wrist-worn processing system. This system features a connector that receives the cable, and a processor that receives digital signals from the cable and collectively processes them with a multi-component algorithm to determine RR. In embodiments, the sensor module digitally filters the IP and ACC waveforms before they pass through the cable. The cable can also include one or more embedded accelerometers, and is configured to attach to the patient's arm.

In all embodiments, the wrist-worn transceiver can include a display configured to render the patient's RR and other vital signs, along with a touchpanel interface. A wireless transceiver within the wrist-worn transceiver can transmit information to a remote computer using conventional protocols such as 802.11, 802.15.4, and cellular (e.g. CDMA or GSM). The remote computer, for example, can be connected to a hospital network. It can also be a portable computer, such as a tablet computer, personal digital assistant, or cellular phone.

Many advantages are associated with this invention. In general, it provides an accurate measurement of RR, along with an independent measurement of a patient's posture, activity level, and motion. These parameters can be collectively analyzed to monitor a hospitalized patient and improve true positive alarms while reducing the occurrence of false positive alarms. Additionally, the measurement of RR is performed with a body-worn monitor that is comfortable, lightweight, and low-profile, making it particularly well suited for ambulatory patients. Such a monitor could continuously monitor a patient as, for example, they transition from the emergency department to the ICU, and ultimately to the home after hospitalization.

Still other embodiments are found in the following detailed description of the invention and in the claims.

BRIEF DESCRIPTION OF THE DRAWINGS

Fig. 1 shows a schematic view of a patient wearing a sensor module on their chest that connects to three electrodes arranged in an Einthoven's triangle configuration and measures both ACC and IP waveforms;

Fig. 2 shows a schematic view of a patient wearing a sensor module on their belly that connects to three electrodes arranged in an Einthoven's triangle configuration and measures both ACC and IP waveforms;

Fig. 3A is a schematic view of a patient wearing an alternate embodiment of the invention featuring a sensor module for measuring IP and ACC waveforms that connects directly through an electrode to the patient's belly;

Fig. 3B is a schematic, cross-sectional view of the sensor module of Fig. 3A connected to the patient's belly with the electrode;

Figs. 4A and 4B show, respectively, a three-dimensional image of the body-worn monitor of the invention attached to a patient during and after an initial indexing measurement;

Fig. 5 shows a three-dimensional image of the wrist-worn transceiver used with the body-worn monitor from Figs. 4A and 4B;

Fig. 6 shows a schematic view of a multi-component algorithm used to collectively process ACC and IP waveforms to measure RR according to the invention;

Fig. 7 shows a schematic drawing of Algorithm 1 used in the multi-component algorithm of Fig. 6;

Fig. 8 shows a schematic drawing of computation steps used in Algorithm 1 to calculate RR;

Fig. 9 shows a series of time-dependent IP waveforms (left-hand side) and their corresponding mathematical derivatives (right-hand side) measured from a slowly breathing patient and processed with digital filters featuring gradually decreasing passbands;

Fig. 10 shows a series of time-dependent IP waveforms (left-hand side) and their corresponding mathematical derivatives (right-hand side) measured from a rapidly breathing patient and processed with digital filters featuring gradually decreasing passbands;

Fig. 11 shows a schematic drawing of Algorithm 2 used in the multi-component algorithm of Fig. 6 to calculate RR;

Fig. 12 shows a schematic drawing of Algorithm 3 used in the multi-component algorithm of Fig. 6 to calculate RR;

Fig. 13 shows a schematic drawing of computation steps used in Algorithms 2 and 3 to calculate RR;

Fig. 14 shows a schematic drawing of a flow chart of computation steps used to calculate coefficients for adaptive filtering which are used in Algorithms 2 and 3 to calculate RR;

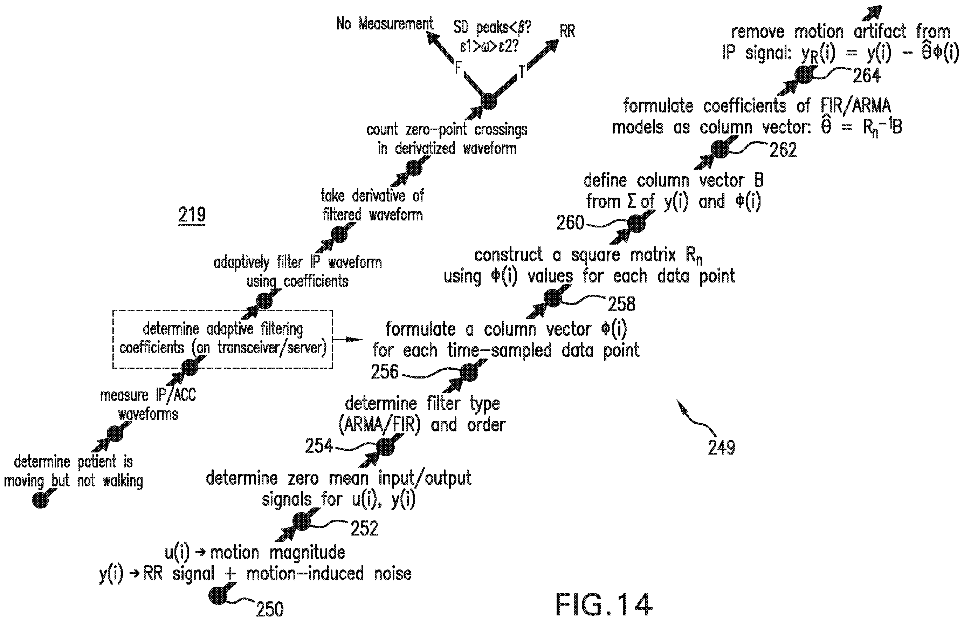

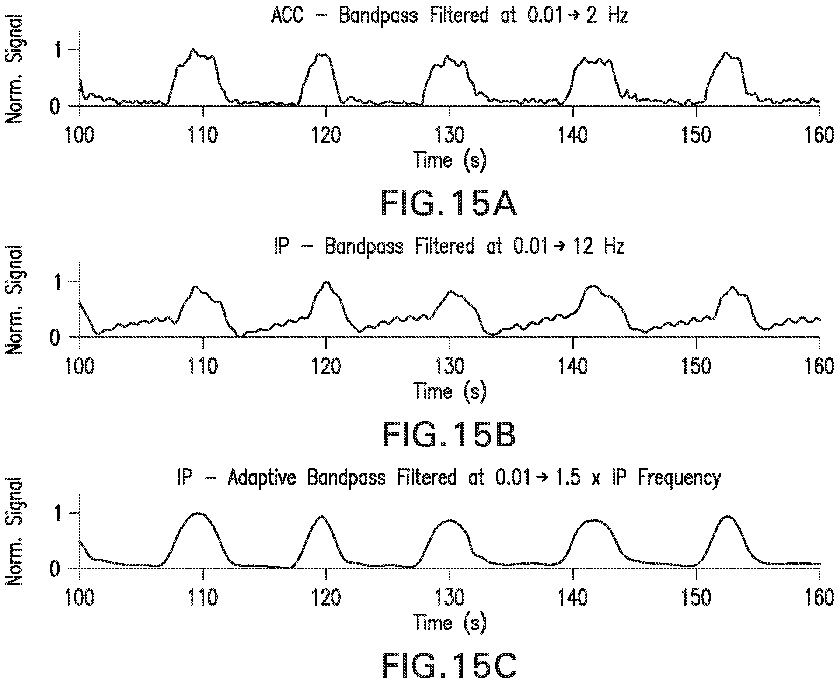

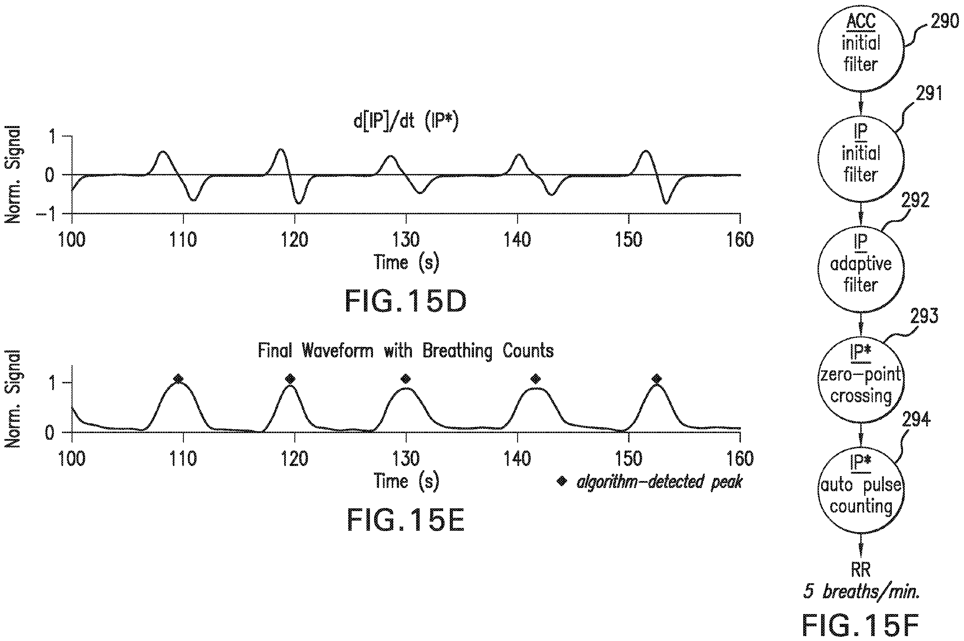

Figs. 15A-E show graphs of an ACC waveform filtered initially with a 0.01 → 2 Hz bandpass filter (Fig. 15A; top), an IP waveform filtered initially with a 0.01 → 12 Hz bandpass (Fig. 15B), an IP waveform adaptively filtered with a bandpass filter ranging from 0.01 Hz to 1.5 times the breathing rate calculated from the ACC waveform in Fig. 15A (Fig. 15C), a first derivative of the filtered IP waveform in Fig. 15C (Fig. 15D), and the adaptively filtered IP waveform in Fig. 15C along with markers indicating slow, deep breaths as determined from the algorithm shown by the flow chart in Fig. 14 (Fig. 15E; bottom);

Fig. 15F is a flow chart showing the algorithmic steps used to process the waveforms shown in Figs. 15A-E;

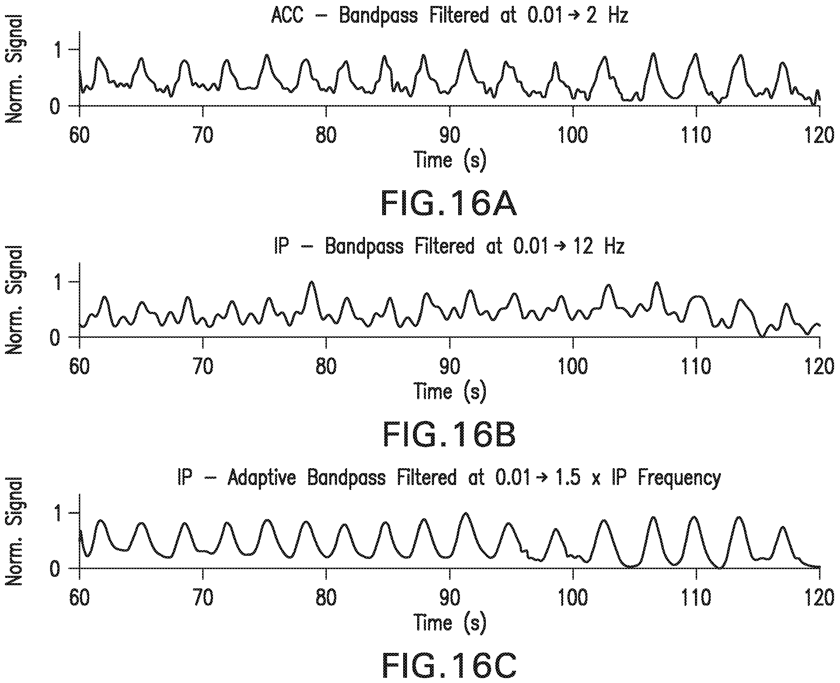

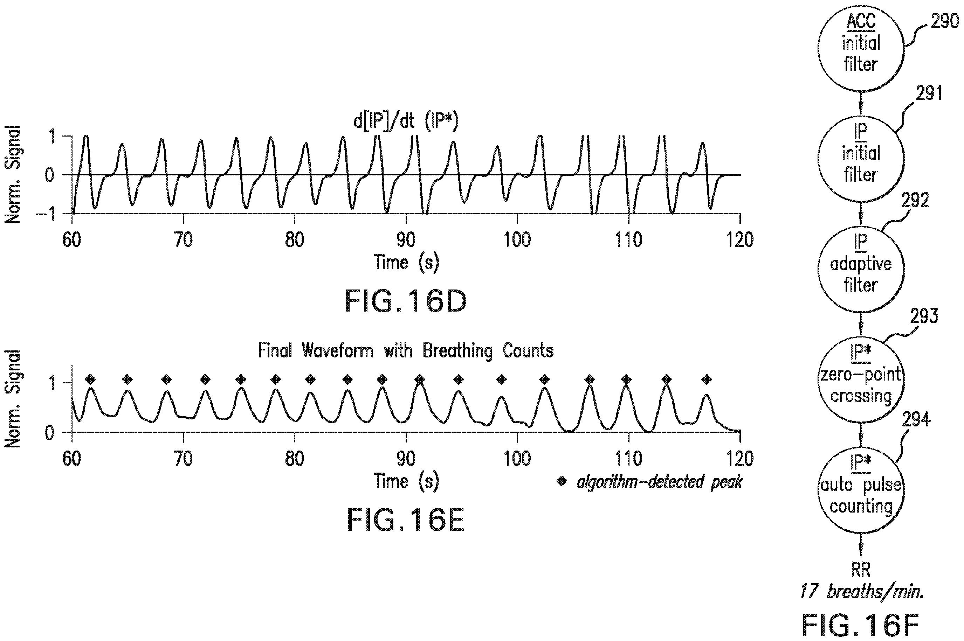

Figs. 16A-E show graphs of an ACC waveform filtered initially with a 0.01 → 2 Hz bandpass filter (Fig. 16A; top), an IP waveform filtered initially with a 0.01 → 12 Hz bandpass (Fig. 16B), an IP waveform adaptively filtered with a bandpass filter ranging from 0.01 Hz to 1.5 times the breathing rate calculated from the ACC waveform in Fig. 16A (Fig. 16C), a first derivative of the filtered IP waveform in Fig. 16C (Fig. 16D), and the adaptively filtered IP waveform in Fig. 16C along with markers indicating fast, deep breaths as determined from the algorithm shown by the flow chart in Fig. 14 (Fig. 16E; bottom);

Fig. 16F is a flow chart showing the algorithmic steps used to process the waveforms shown in Figs. 16A-E;

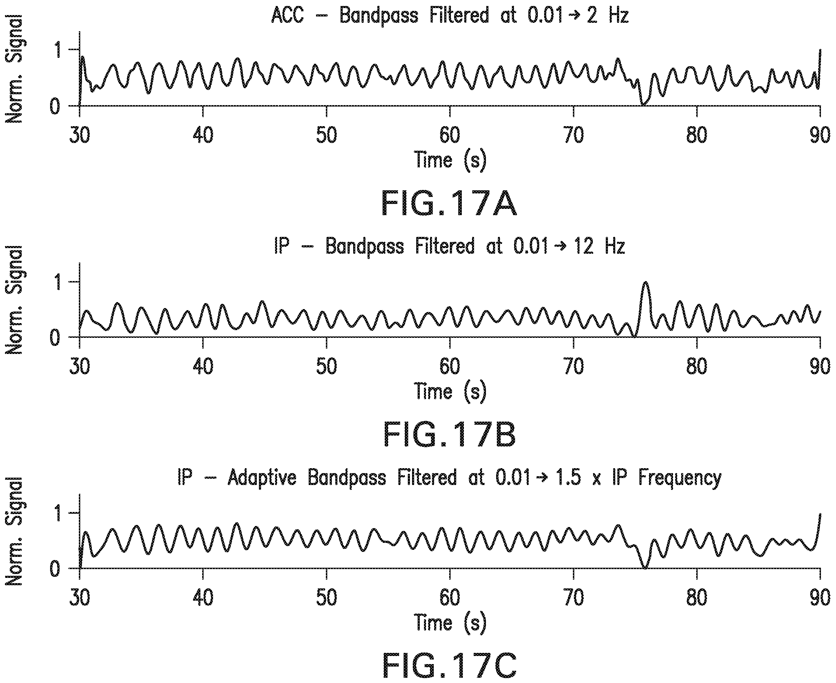

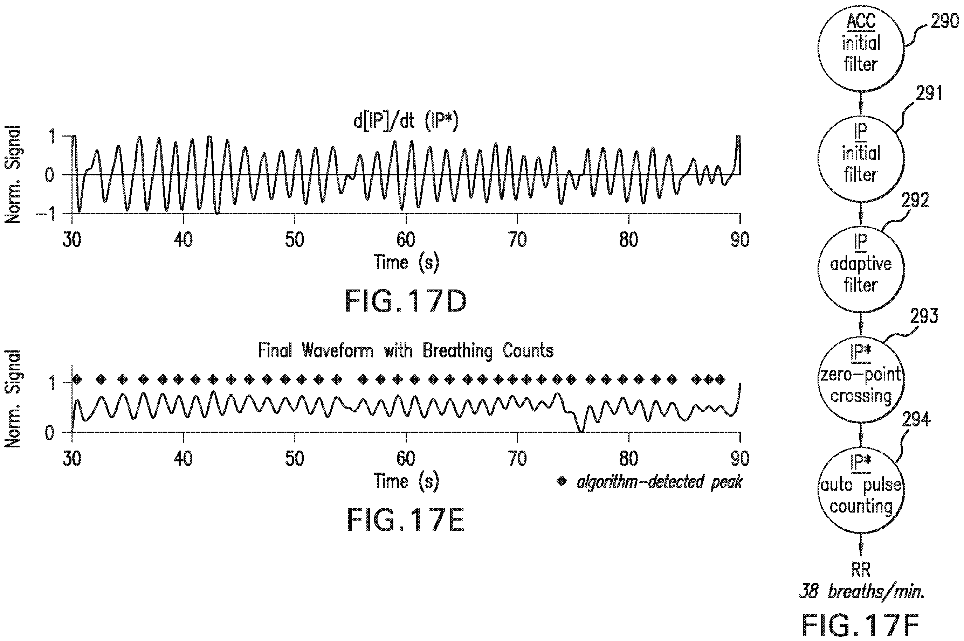

Figs. 17A-E show graphs of an ACC waveform filtered initially with a 0.01 → 2 Hz bandpass filter (Fig. 17A; top), an IP waveform filtered initially with a 0.01 → 12 Hz bandpass (Fig. 17B), an IP waveform adaptively filtered with a bandpass filter ranging from 0.01 Hz to 1.5 times the breathing rate calculated from the ACC waveform in Fig. 17A (Fig. 17C), a first derivative of the filtered IP waveform in Fig. 17C (Fig. 17D), and the adaptively filtered IP waveform in Fig. 17C along with markers indicating very fast, deep breaths as determined from the algorithm shown by the flow chart in Fig. 14 (Fig. 17E; bottom);

Fig. 17F is a flow chart showing the algorithmic steps used to process the waveforms shown in Figs. 17A-E;

Fig. 18 shows a schematic drawing of Algorithm 4 used in the multi-component algorithm of Fig. 6 to calculate RR;

Fig. 19 shows a schematic drawing of computation steps used in Algorithm 4 to calculate RR;

Figs. 20A-F show, respectively, a time-domain IP waveform measured from a running patient (Fig. 20A), a time-domain ACC waveform simultaneously measured from the same patient (Fig. 20B), a frequency-domain power spectrum of the IP waveform of Fig. 20A (Fig. 20C), a frequency-domain power spectrum of the ACC waveform of Fig. 20B (Fig. 20D), the frequency-domain power spectrum of the IP waveform of Fig. 20C processed with a notch filter (Fig. 20E), and the frequency-domain power spectrum of the IP waveform of Fig. 20E processed with a smoothing filter (Fig. 20F);

Figs. 21A-F show, respectively, a time-domain IP waveform measured from a walking patient (Fig. 21A), a time-domain ACC waveform simultaneously measured from the same patient (Fig. 21B), a frequency-domain power spectrum of the IP waveform of Fig. 21A (Fig. 21C), a frequency-domain power spectrum of the ACC waveform of Fig. 21B (Fig. 21D), the frequency-domain power spectrum of the IP waveform of Fig. 21C processed with a notch filter (Fig. 21E), and the frequency-domain power spectrum of the IP waveform of Fig. 21E processed with a smoothing filter (Fig. 21F);

Figs. 22A-C show, respectively, a time-domain IP waveform measured from a stationary patient laying down on their back and breathing normally (Fig. 22A), a time-domain ACC waveform measured simultaneously from the same patient (Fig. 22B), and frequency-domain power spectra of both the time-domain IP waveform of Fig. 22A and ACC waveform of Fig. 22B (Fig. 22C);

Figs. 23A-C show, respectively, a time-domain IP waveform measured from a stationary patient laying down on their back and breathing rapidly (Fig. 23A), a time-domain ACC waveform measured simultaneously from the same patient (Fig. 23B), and frequency-domain power spectra of both the time-domain IP waveform of Fig. 23A and ACC waveform of Fig. 23B (Fig. 23C);

Figs. 24A-C show, respectively, a time-domain IP waveform measured from a stationary patient laying face down and breathing normally (Fig. 24A), a time-domain ACC waveform measured simultaneously from the same patient (Fig. 24B), and frequency-domain power spectra of both the time-domain IP waveform of Fig. 24A and ACC waveform of Fig. 24B (Fig. 24C);

Figs. 25A-E show frequency-domain power spectra of, respectively, an IP waveform processed with no smoothing filter (Fig. 25A), a 5.0 Hz smoothing filter (Fig. 25B), a 2.5 Hz smoothing filter (Fig. 25C), a 1.0 Hz smoothing filter (Fig. 25D), and a 0.5 Hz smoothing filter (Fig. 25E);

Figs. 26A-E show frequency-domain power spectra of, respectively, an IP waveform processed with no running average (Fig. 26A), a 10-point running average (Fig. 26B), a 20-point running average (Fig. 26C), a 50-point running average (Fig. 26D), and a 100-point running average (Fig. 25E);

Fig. 27A shows a time-domain IP waveform measured from a rapidly breathing stationary patient;

Figs. 27B-D show frequency-domain power spectra calculated from the time-domain IP waveform of Fig. 27A using, respectively, a 1000-point FFT, a 500-point FFT, and a 250-point FFT;

Fig. 28A shows a time-domain IP waveform measured from a slowly breathing stationary patient;

Figs. 28B-D show frequency-domain power spectra calculated from the time-domain IP waveform of Fig. 28A using, respectively, a 1000-point FFT, a 500-point FFT, and a 250-point FFT;

Fig. 29 shows a schematic view of the patient of Fig. 1 and a coordinate axis used with an algorithm and ACC waveforms to determine the patient's posture;

Fig. 30A shows a graph of time-dependent ACC waveforms measured from a patient's chest during different postures; and

Fig. 30B shows a graph of time-dependent postures determined by processing the ACC waveforms of Fig. 30A with an algorithm and coordinate axis shown in Fig. 29.

## DETAILED DESCRIPTION OF THE INVENTION

### Sensor Configuration

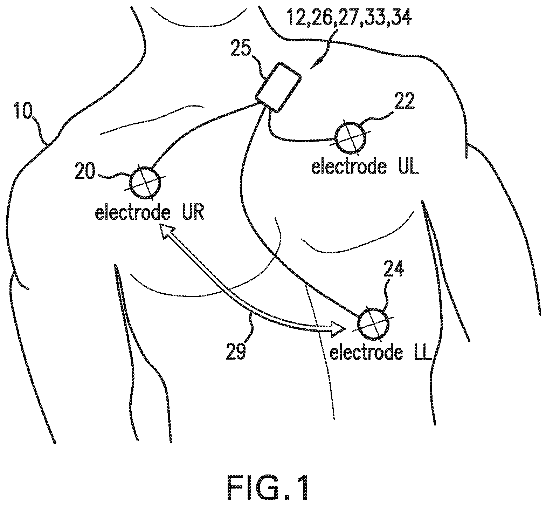

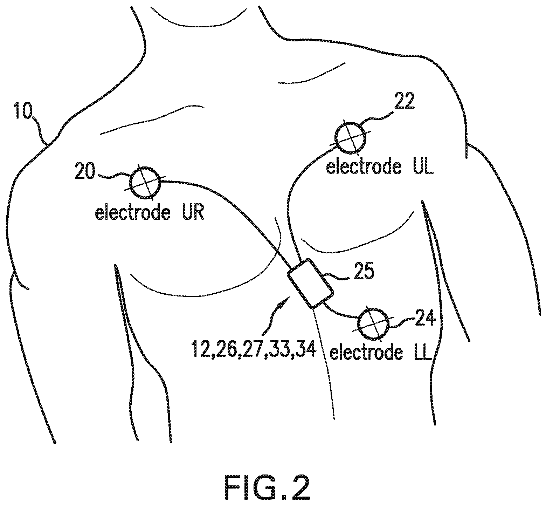

Referring to Figs. 1 and 2, a sensor module 25 featuring an IP circuit 27 and accelerometer 12 is mounted on the chest of a patient 10 to simultaneously measure IP and ACC waveforms. A multi-component algorithm, featuring specific algorithms based on simple peak counting, FFT analysis, and adaptive filters processes these waveforms to accurately measure RR even when the patient 10 is moving. During a measurement, both the IP 27 and an ECG circuit 26 within the sensor module connect to a trio of electrodes 20, 22, 24 typically positioned on the patient's torso in an Einthoven's triangle configuration. Each electrode 20, 22, 24 measures a unique analog signal that passes through a shielded cable to the ECG circuit 26. This component typically includes a differential amplifier and a series of analog filters with passbands that pass the high and low-frequency components that contribute to the ECG waveform, but filter out components associated with electrical and mechanical noise. To determine RR, the IP circuit 27 generates a low-amperage current (typically 1-4 mA) that is modulated at a high frequency (typically 50-100 kHz). The current typically passes through electrode LL ('lower left') 24, which is located on the lower left-hand side of the patient's torso. It then propagates through the patient's chest, as indicated by the arrow 29, where a respiration-induced capacitance change modulates it according to the RR. Electrode UR ('upper right') 20 detects the resultant analog signal, which is then processed with a separate differential amplifier and series of analog filters within the IP circuit to determine an analog IP waveform featuring a low-frequency series of pulses corresponding to RR. Typically the analog filters in the IP circuit 27 are chosen to filter out high-frequency components that contribute to the ECG QRS complex.

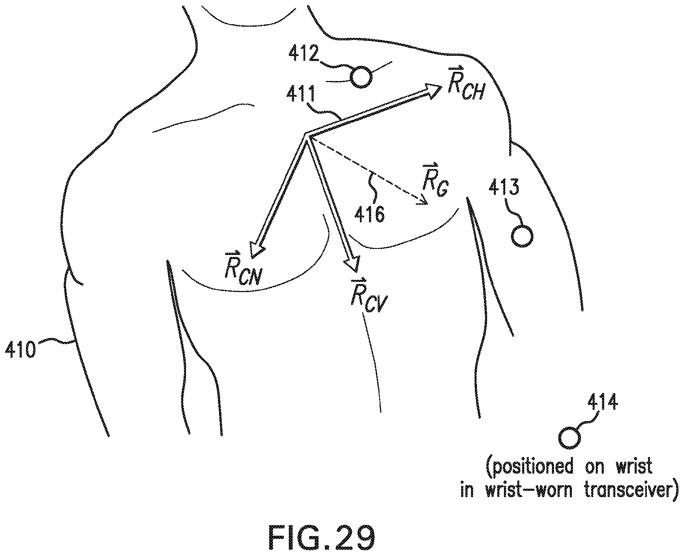

The accelerometer 12 mounted within the sensor module 25 measures ACC waveforms that are modulated by the patient's general motion and posture, along with small respiratory-induced motions of the patient's torso. The accelerometer 12 simultaneously measures acceleration (e.g. motion) along x, y, and z axes of a local coordinate system, such as that shown in Fig. 29. As shown in this figure, and described in more detail below, the accelerometer 12 is preferably aligned so the z axis points into the patient's torso. Within the accelerometer 12 is an internal analog-to-digital converter that generates a digital ACC waveform corresponding to each axis.

Also within the sensor module 25 is a microprocessor 33 and analog-to-digital converter (not shown in the figure) that digitize the IP and ACC waveforms, and sends them through a serial protocol (e.g. the control area network, or CAN protocol) to the wrist-worn transceiver for further processing. There, IP and ACC waveforms are processed with the multi-component algorithm to determine the patient's RR. Alternatively, the algorithms can be performed in part with a remote server, or with the microprocessor 33 mounted within the sensor module. Additional properties such as the patient's posture, degree of motion, and activity level are determined from these same digital ACC waveforms. The axis within the accelerometer's coordinate system that is aligned along the patient's torso (and thus orthogonal to their respiration-induced torso movement) is typically more sensitive to events not related to respiration, e.g. walking and falling.

In a preferred embodiment, digital accelerometers manufactured by Analog Devices (e.g. the ADXL345 component) are used in the configuration shown in Fig. 1. These sensors detect acceleration over a range of +/-2 g (or, alternatively, up to +/-8 g) with a small-scale, low-power circuit.

Many patient's are classified as 'belly breathers', meaning during respiration their belly undergoes larger movements than their chest. A relative minority of patients are 'chest breathers', indicating that it is the chest that undergoes the larger movements. For this reason it is preferred that RR is determined using an ACC waveform detected along the z-axis with an accelerometer positioned on the patient's belly. In alternate configurations, a separate accelerometer mounted on the chest can be used in its place or to augment data collected with the belly-mounted sensor. Typically, ACC waveforms along multiple axes (e.g. the x and y-axes) are also modulated by breathing patterns, and can thus be used to estimate RR. In still other configurations multiple signals from one or more accelerometers are collectively processed to determine a single 'effective' ACC waveform representing, e.g., an average of multiple ACC waveforms. This waveform is then processed as described herein to determine the patient's RR.

In other embodiments, the sensor module 25 includes a temperature sensor 34, such as a conventional thermocouple, that measures the skin temperature of the patient's chest. This temperature is typically a few degrees lower than conventional core temperature, usually measured with a thermometer inserted in the patient's throat or rectum. Despite this discrepancy, skin temperature measured with the temperature sensor 34 can be monitored continuously and can therefore be used along with RR and other vital signs to predict patient decompensation.

In a preferred embodiment, both the ECG and IP waveforms are generated with a single ASIC, or alternatively with a circuit composed of a series of discrete elements which are known in the art. The ASIC has an advantage in that it is a single chip and is included in a circuit that typically contains fewer electrical components, is relatively small, and is typically very power efficient. In either embodiment, the ECG circuit typically includes an internal analog-to-digital converter that digitizes both waveforms before transmission to the wrist-worn transceiver for further processing.

Transmission of digital IP, ECG, and ACC waveforms, along with processed RR values, has several advantages over transmission of analog waveforms. First, a single transmission line in the monitor's cabling can transmit multiple digital waveforms, each generated by different sensors. This includes multiple ECG waveforms (corresponding, e.g., to vectors associated with three, five, and twelve-lead ECG systems) from the ECG circuit 26, the IP waveform from the IP circuit 27, and ACC waveforms associated with the x, y, and z axes of accelerometers 10, 12 attached to the patient's chest. Limiting the transmission line to a single cable reduces the number of wires attached to the patient, thereby decreasing the weight and cable-related clutter of the body-worn monitor. Second, cable motion induced by an ambulatory patient can change the electrical properties (e.g. electrical impendence) of its internal wires. This, in turn, can add noise to an analog signal and ultimately the vital sign calculated from it. A digital signal, in contrast, is relatively immune to such motion-induced artifacts. More sophisticated ECG circuits can plug into the wrist-worn transceiver to replace the three-lead system shown in Figs. 1 and 2. These ECG circuits, for example, can include, e.g., five and twelve leads.

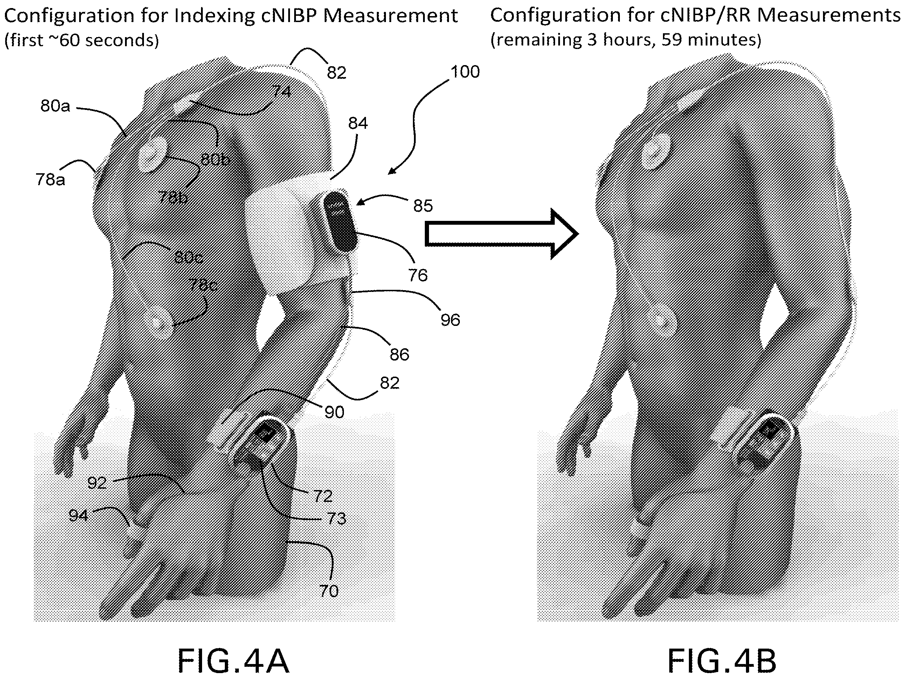

Digital data streams are typically transmitted to the wrist-worn transceiver using a serial protocol, such as the CAN protocol, USB protocol, or RS-232 protocol. CAN is the preferred protocol for the body-worn monitor described in Figs. 4A, 4B.

Multi-Component Algorithm for Determining RR

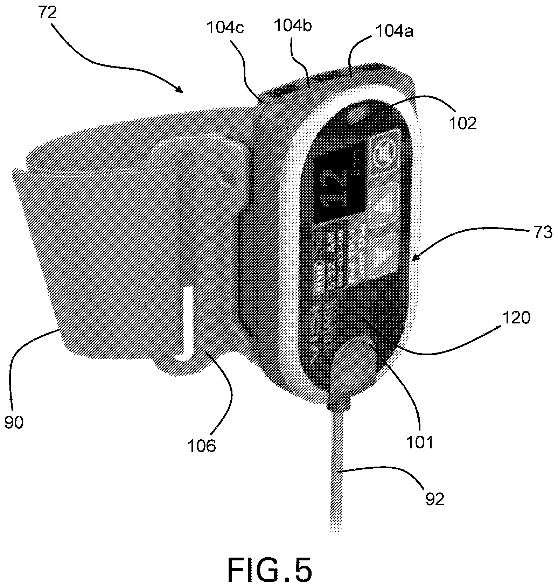

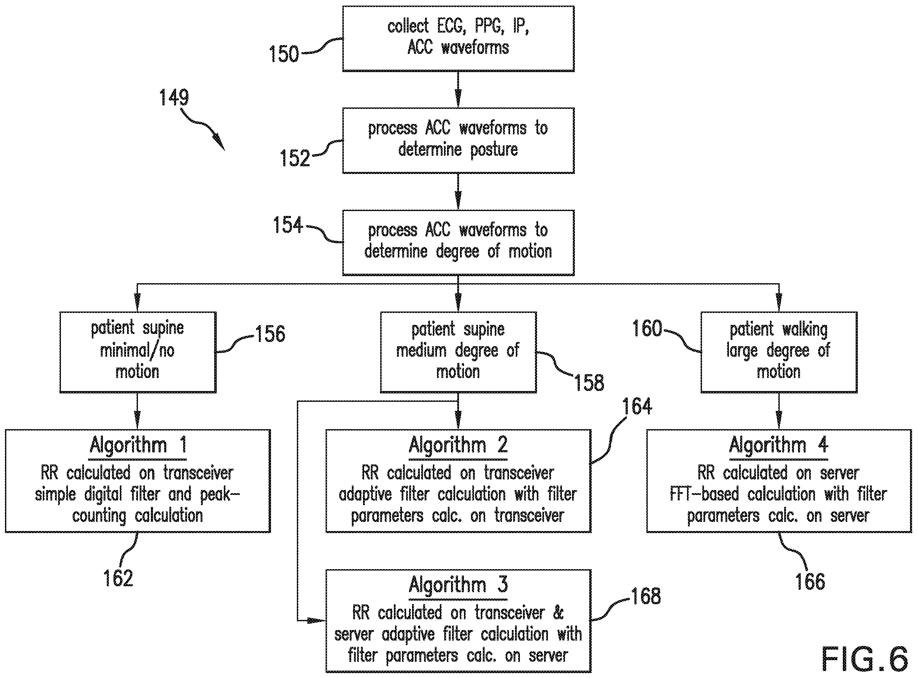

Fig. 6 shows an overview of a multi-component algorithm 149, implemented with the body-worn monitor shown in Figs. 4A, 4B, and 5 and an accompanying server, which determines a patient's RR according to the invention. The algorithm features the following components for determining RR from a hospitalized patient undergoing different levels of motion:

**Algorithm 1 -** simple peak-counting calculation implemented on the wrist-worn transceiver; used when patient motion is minimal or non-existent
**Algorithm 2** - adaptive filtering calculation with filtering parameters calculated on the wrist-worn transceiver; used when some patient motion is evident
**Algorithm 3** - adaptive filtering calculation with filtering parameters calculated on the server and then transmitted to the wrist-worn transceiver; used when some patient motion is evident
**Algorithm 4** - FFT-based calculation with active noise cancellation, performed on the server with processed data transmitted to the wrist-worn transceiver; used when large amounts of patient motion is evident

Each of these algorithms, along with both respiratory and motion data to support them, are described in more detail below.

Algorithms 1-4 - Simultaneous Determination of Motion and Respiratory Signals

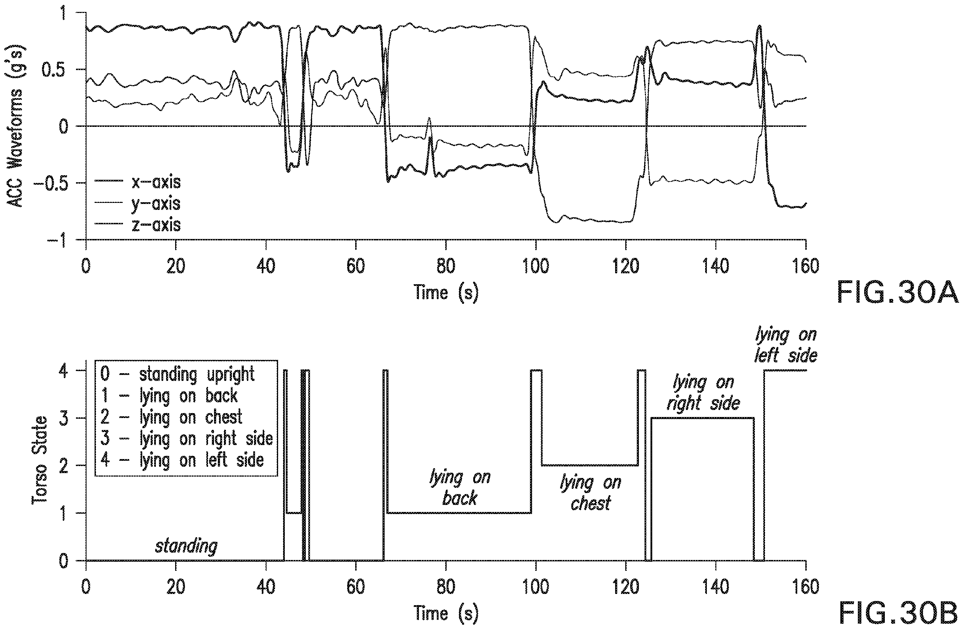

Referring again to Fig. 6, before Algorithms 1-4 are implemented, the body-worn monitor collects ECP, PPG, IP, and ACC waveforms (step 150) from the patient, as described above with references to Figs. 1-5. A patient's degree of motion (step 152) and their posture (step 154) are determined by processing the ACC waveforms using algorithms described in detail in the following pending patent applications: BODY-WORN MONITOR FEATURING ALARM SYSTEM THAT PROCESSES A PATIENT'S MOTION AND VITAL SIGNS (U.S.S.N 12/469,182; filed May 20, 2009) and BODY-WORN VITAL SIGN MONITOR WITH SYSTEM FOR DETECTING AND ANALYZING MOTION (U.S.S.N 12/469,094; filed May 20, 2009). Figs. 29 and 30, below, further indicate how processing of ACC waveforms yields both posture, degree of motion, and activity level.
The multi-component algorithm 149 processes these motion-related properties to determine which of the four above-described algorithms to implement. The selected algorithm then simultaneously processes ECG and ACC waveforms to determine RR. As described above, motion can significantly complicate determination of RR, as these two signals often occur at similar frequencies (typically 0.1-2 Hz), and feature signal components (e.g. 'pulses' due to breathing and walking) that look similar and themselves are composed of common frequency components. In addition to RR, the body-worn monitor calculates cNIBP using the ECG and PPG waveforms, and SpO2 from PPG waveforms generated simultaneously with both red and infrared light sources, as described above. HR is calculated from the ECG waveform using methods known in the art.
Both high-level and detailed aspects of Algorithms 1-4 are described below.

Algorithm 1 - Peak Counting

Algorithm 1 (step 162) is implemented after determining that the patient is supine and undergoing minimal or no motion (step 156). Here, it is assumed that the IP waveform used to determine RR is not significantly corrupted by motion, and thus RR can be calculated with relatively simple means. Put another way, in this case there is minimal coupling between the ACC and IP waveforms; collective processing of these waveforms to remove motion-related artifacts, as is done with Algorithms 2-4, is therefore not required. Algorithm 1 typically yields a highly accurate RR, and because of its simplicity requires relatively few computational cycles. This in turn reduces power consumption and prolongs battery lifetime on the wrist-worn transceiver.

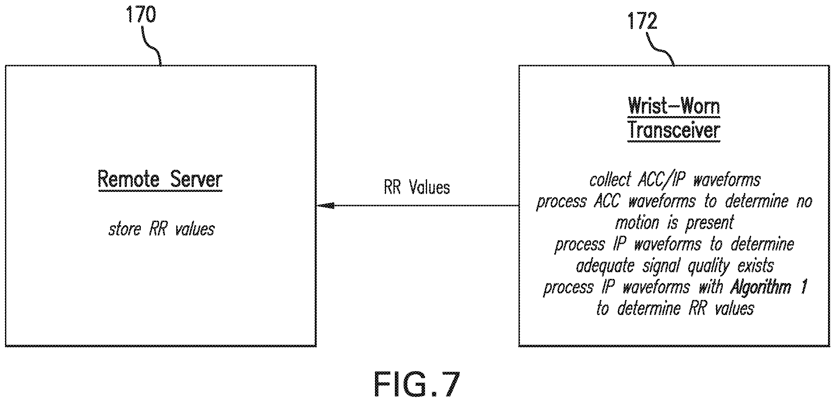

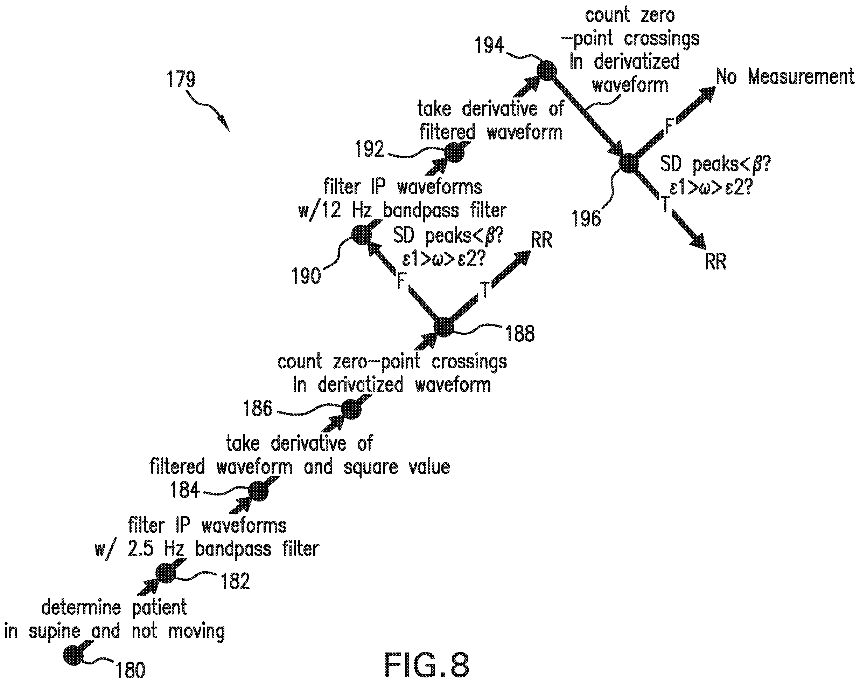

Fig. 7 shows a high-level diagram describing Algorithm 1. In this case, all calculations are performed with a microprocessor on the wrist-worn transceiver 172 to yield final values for RR. Once determined, a wireless system on the transceiver sends these values to a remote server 170 for further processing, display, storage, and incorporation into a hospital's medical records system. The wrist-worn transceiver 172 additionally displays RR values on the transceiver's touchpanel display so that they can be viewed at the patient's bedside. Fig. 8 shows the specific details of this calculation. It begins by confirming that the patient is supine and not moving (step 180), which as described above is accomplished by processing ACC signals generated by the three 3-axis accelerometers integrated in the body-worn monitor. The IP waveform is then digitally filtered with a 2.5 Hz digital bandpass filter to remove extraneous noise (typically from electrical and mechanical sources) that complicates processing of the waveform. In typical applications, the digital filter features a second-order infinite impulse response (IIR). In order to remove any phase distortion, the IIR filter is executed in both the forward and reverse directions. The reverse filtering step doubles the effective order of the filter, and cancels out any phase distortion introduced by the forward filtering operation. The reverse filtering step is implemented by executing the standard IIR difference equation, performing a time-reversal on the outputted data, and then executing the same IIR difference equation. While effective in removing phase distortion, such additional steps require an extra difference computation which cannot be performed in real-time on a stream of data. This, in turn, increases power consumption in the wrist-worn transceiver, and thus shortens battery life.

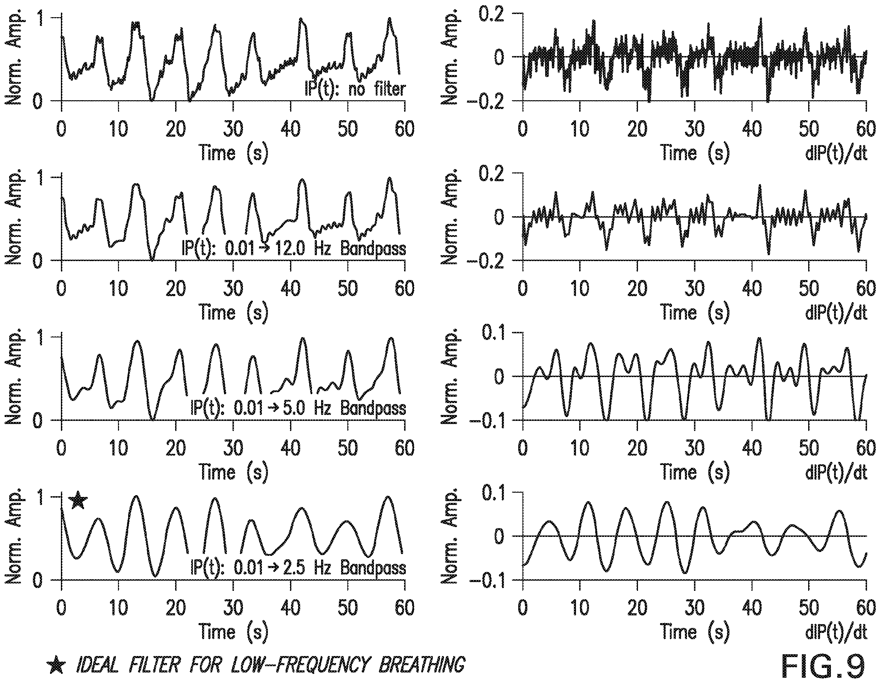

Fig. 9 shows how filtering raw IP waveforms with different passbands ultimately affects the signal-to-noise ratio of these signals and their corresponding derivatives. Ultimately, it is these derivatives that are processed to determine RR.

Referring again to Fig. 8, the digitally filtered IP waveform is then derivatized and squared using Algorithm 1 (step 184), yielding a signal similar to that shown in the right-hand side of Fig. 9. Taking a derivative removes any low-frequency baseline components from the IP waveform, and additionally generates a clear, well-defined zero-point crossing corresponding to each peak in the IP signal. Each peak corresponds to each respiration event. The derivative used for this calculation is typically a 5-point derivative, meaning that data point $IPP_{xi+5}$ is subtracted from data point $IPP_{xi}$ to calculate the derivative. When the IP waveform is sampled at 50 Hz, which is preferred, this means data points separated by 0.1 seconds are used for the derivative. Such a method for taking a derivative is preferred over that using directly sequential data points, i.e. a derivative where data point $IPP_{xi+1}$ is subtracted from data point $IPP_x$ (i.e. the data points are separated by 0.02 seconds for data collection rates of 50 Hz). A 5-point derivative typically yields a relatively strong signal featuring a high signal-to-noise ratio and minimal phase distortion. Additionally, as shown in Fig. 9, the passband of the digital filter has a significant impact on the derivatized signal, and features an optimal value that is closely coupled to the actual value of RR. For example, for the signals shown in Fig. 9 (corresponding to roughly 8 breaths/minute), the ideal high-frequency cutoff for the passband is near 2.5 Hz, as indicated in the figure with a star. This yields a signal where the respiratory-induced peaks can be easily processed by counting the zero-point crossing with a counting algorithm (step 186). Once determined, this initial guess at RR, along with the derivatized signal used to determine it, is compared to a series of pre-determined metrics to estimate the accuracy of the determination (step 188). In particular, the number of peaks determined during a pre-determined time period (e.g. 20 seconds) is then compared over three consecutive periods. The standard deviation (SD in Fig. 8) of the counts within these periods is then calculated using standard means, and then compared to a pre-determined value ($\beta$) to ensure that the RR is relatively constant during the measurement period. A low standard deviation, for example, would indicate that the RR is relatively constant for the three consecutive 20-second periods, which in turns indicates that the measurement is likely accurate. In contrast, a high standard deviation indicates that the RR is either changing rapidly or characterized by a waveform having a low signal-to-noise ratio, which in turn indicates that the measurement is likely inaccurate. In typical cases, $\beta$ has a value between 0-2 breaths/minute. The actual value of RR ($\omega$ in Fig. 8) is then compared to pre-determined threshold values ($\epsilon1$ and $\epsilon2$) to estimate if it is realistic. For example, $\epsilon1$ represents an upper level of RR, and is typically 60 breaths/minute. At this level a patient may be hyperventilating. $\epsilon2$ represents a lower level of RR, and is typically about 5 breaths/minute. Below this and a patient may be undergoing respiratory failure.

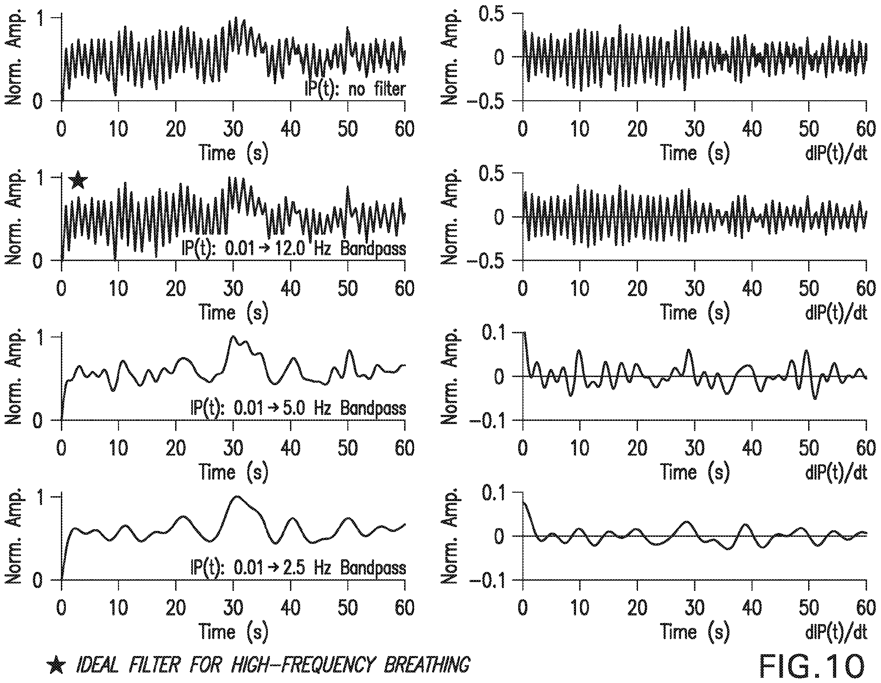

If the RR value calculated during steps 180-186 meets the criteria defined in step 188, it is considered to be valid, or 'true' (T in Fig. 8), and is reported by the body-worn monitor. In contrast, if the RR value fails the criteria defined in step 188, it is assumed to be 'false' (F in Fig. 8), and further processing is performed. In particular, the raw IP waveform is processed again with a digital bandpass filter characterized by a different passband (step 190) which is typically 12 Hz. The second filter may yield a waveform featuring high-frequency components that are removed by the first filter. After this filtering step the calculations originally performed during steps 184, 186, 188 are repeated. Fig. 10 indicates the merits of processing the raw IP waveform with a digital filter having an increase passband. In this case, the patient is undergoing a high RR (roughly 60 breaths/minute), with each sharp pulse in the IP waveform composed of a large and far-ranging collection of frequency components. Thus, filtering this waveform with the 2.5 Hz digital filter described in step 182 and shown in the lower portion of Fig. 10 yields a filtered IP waveform wherein the breathing-induced pulses are depleted. Taking the derivative of this signal yields the waveform shown in the lower right-hand portion of Fig. 10. The waveform lacks the information to properly determine RR. In contrast, as indicated in Fig. 10 by a star, digitally filtering the raw IP waveform with a 12 Hz passband yields a relatively noise-free signal from which a derivatized waveform can be determined as described above (step 192). From this waveform, zero-point crossings, each corresponding to an individual breath, can be easily counted as used to estimate a value of RR (step 194). This value is then compared to the same pre-determined values ($\beta$, $\epsilon1$, $\epsilon2$) used during step 188 to estimate the validity of the calculated RR (step 196). If determined to be accurate, this value is reported by the body-worn monitor as described above; if not, the algorithm determines that an accurate measurement cannot be made, a series of dashes (e.g. '---') are rendered by the monitor, and the process is repeated.

Algorithms 2 and 3 - Adaptive Filtering

Both Algorithms 2 and 3 describe methods for determining RR from ACC and IP waveforms using a technique called 'adaptive filtering'. The general premise of adaptive filtering, as used in this application, is that motion-induced frequency components in the ACC waveform are determined and then filtered from the simultaneously measured IP waveform. This yields a clean, relatively uncorrupted waveform from which RR can be accurately determined. In Algorithm 2, the coefficients for adaptive filtering are determined from the ACC waveform by processing performed on the wrist-worn transceiver. This may be computationally 'expensive', and thus increase power consumption, but has the added benefit that all calculations can be done in the absence of a remote server. In Algorithm 3, both IP and ACC waveforms are transmitted to the remote server following a measurement, and the coefficients for adaptive filtering are calculated on this platform. Afterwards, they are sent back to the wrist-worn transceiver, where the calculation is completed. Alternatively, the waveforms are fully processed on the remote server with adaptive filtering, and values of RR are transmitted back to the wrist-worn transceiver. In both cases, once received, the values of RR are displayed and availed for any follow-on alarming/alerting applications.

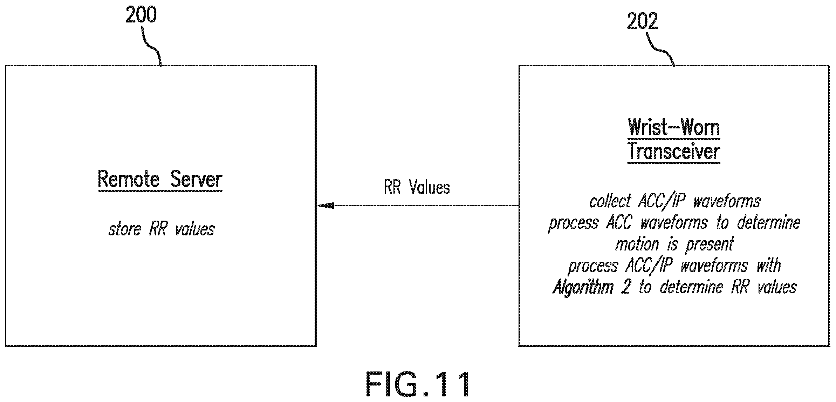

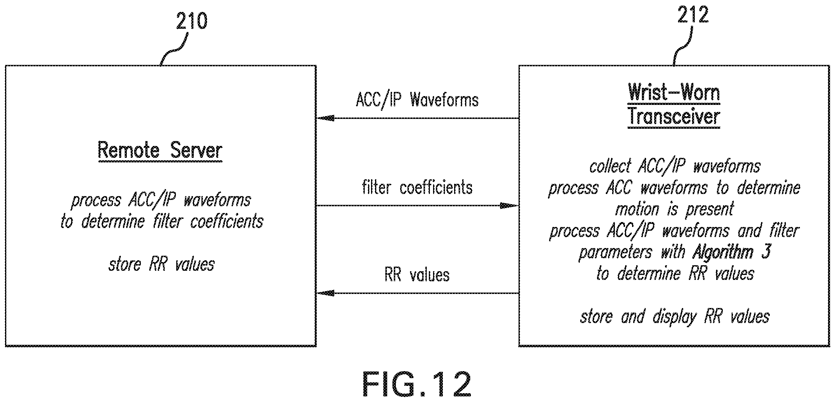

Fig. 11 provides a high-level overview of Algorithm 2, which features a wrist-worn transceiver 202 that collects ACC and IP waveforms, processes the IP waveforms to determine that motion is present, and then collectively processes the ACC and IP waveforms with an adaptive filter algorithm to determine RR. Once calculated, this parameter is wirelessly transmitted to a remote server 200 for storage and further processing. During Algorithm 3, shown schematically in Fig. 12, the wrist-worn transceiver 212 collects ACC and IP waveforms, and wirelessly transmits these to the remote server 210 for processing. The remote server determines adaptive filter parameters as described in detail below, and then wirelessly transmits these back to the transceiver 212, which uses them to digitally filter the IP waveform to determine RR in the presence of motion. Once determined, the value of RR is transmitted from the transceiver 212 to the server 210 for storage and follow-on analysis.

According to Algorithm 3, the coefficients determined by the remote server 210 can be continuously used by the wrist-worn transceiver 212 for an extended, follow-on period to adaptively filter IP waveforms. This further increases computational efficiency and reduces power consumption on the transceiver. Typically the follow-on period is several minutes, and preferably the motion during this period is similar in type and magnitude to that used when the coefficients were originally calculated. This ensures that motion can be adequately filtered out from the IP waveform. If the type or magnitude of the patient's motion changes, both IP and ACC waveforms are retransmitted from the transceiver 212 to the remote server 210, and the process illustrated in Fig. 12 is repeated.

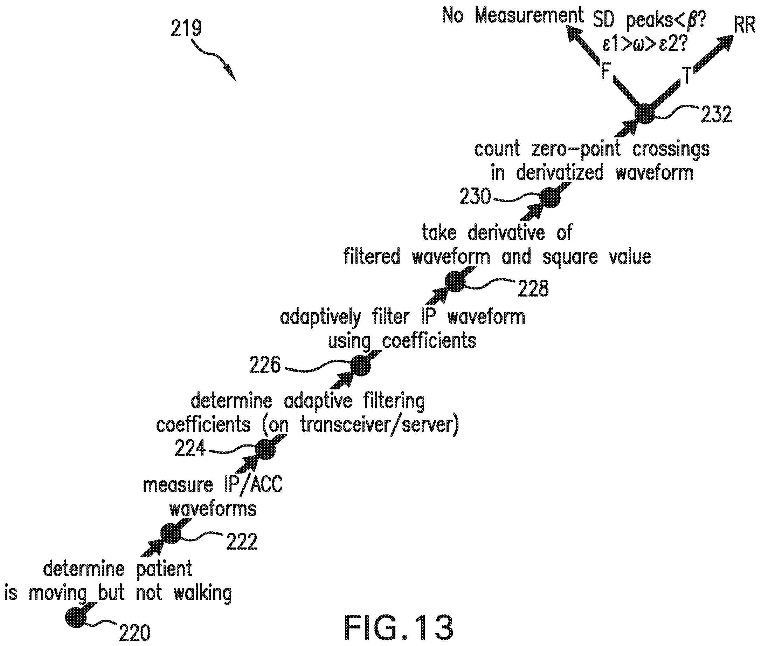

Fig. 13 describes a general algorithm for adaptive filtering which can be used for both Algorithm 2 and/or 3. Both algorithms rely on a 'batch process' technique, which is designed for a linear deterministic system, and uses the ACC waveforms measured from the chest-worn accelerometer (component 12 in Fig. 1) as a reference signal. In alternate embodiments, this approach can be replaced with more sophisticated models, such as those involving recursive methods or non-linear deterministic systems. As described above, both Algorithms 2 and 3 begin with a determination that the patient is moving (e.g. moving their arms or legs), but not walking (step 220). The body-worn monitor then measures ACC and IP waveforms (step 222), and then the adaptive filter coefficients are determined (step 224) on either the wrist-worn transceiver (Algorithm 2) or the remote server (Algorithm 3). Once determined, the coefficients are used to adaptively filter the IP waveform to remove any motion-induced noise (step 226), resulting in a relatively noise-free waveform that is uncorrupted by motion and other noise (e.g. that from electrical and mechanical sources). At this point the waveform is processed in a manner similar to that described with reference to Algorithm 1. Specifically, the waveform is derivatized and squared (step 228) to remove any low-frequency baseline components and generate a zero-point crossing for each respiratory-induced pulse. The algorithm then counts the zero-point crossings (step 230) to determine an initial RR, which is then compared to the pre-determined values ($\beta$, $\varepsilon1$, $\varepsilon2$) described above to estimate if this rate is valid.

Fig. 14 highlights an algorithm 249 for determining the adaptive filtering coefficients, and for performing an adaptive digital filter. Prior to implementing the algorithm, IP and ACC waveforms from the x, y, and z-axes are collected at a frequency of 50 Hz for a period ranging from about 2-3 minutes, resulting in N samples as defined below. During this period it is assumed that the patient is undergoing moderate motion, and that the IP waveform contains a motion artifact.

The noise reference u[i] is defined as the vector length of the chest acceleration, as determined with the accelerometer mounted on the patient's chest, which combines all three axes into a single parameter as given in Eq. 1 below (step 250).

$$u[i] = \sqrt{(acc_{Cx}[i])^2 + \left(acc_{Cy}[i]\right)^2 + (acc_{Cz}[i])^2} \qquad (1)$$

The measured output y[i] is the IP waveform, which contains signals due to both respiration and motion. Note that for this calculation the two waveforms should be sampled at the same rate. If the IP waveform is sampled at a higher frequency than that used for the ACC waveform (e.g. 50 Hz), then this waveform must be converted into a 50 Hz waveform using conventional mathematical techniques, such as decimation or a numerical 'mapping' operation.

At this point zero mean input and output signals for u[i] and y[i] are constructed by subtracting the ensemble signal mean from each signal. This operation, shown below in Eqs. 2 and 3, effectively removes any DC offset (step 252).

$$u[i] = u[i] - \left(\frac{1}{N}\right) \sum_{k=1}^{N} u[k] \qquad (2)$$

$$y[i] = y[i] - \left(\frac{1}{N}\right) \sum_{k=1}^{N} y[k] \qquad (3)$$

A mathematical model used to define the impulse response function of the adaptive filter must be chosen, with the two primary options being filters based on FIR (finite impulse response) or ARMA (autoregressive moving average) models. Both models are indicated below in Eqs. 4 and 5, but only one should be chosen and implemented during the algorithm (step 254):

*FIR Model*

$$H[z] = b_0 + b_1 z^{-1} + \cdots + b_L z^{-L} \qquad (4)$$

*ARMA Model*

$$H[z] = \frac{b_0 + b_1 z^{-1} + \cdots + b_m z^{-m}}{1 + a_1 z^{-1} + \cdots + a_n z^{-p}} \qquad (5)$$

At this point the order of the filter is determined. For the FIR model, the order is L; for the ARMA model, the order is m and p. In a preferred embodiment, the orders for L, m, and p are, respectively, 25, 2, and 2; these values may vary depending on the degree of motion and the computational capabilities on the wrist-worn transceiver. A column vector phi $\varphi$[i] is then formulated for each time-sampled data point for both the FIR and ARMA models, as described in Eqs. 6 and 7 below (step 256). In these equations the superscript T represents the matrix transpose.

*FIR Model*

$$\varphi[i] = [u[i] \quad u[i-1] \quad u[i-2] \quad u[i-3] \quad \cdots \quad u[i-25]]^T \qquad (6)$$

*ARMA Model*

$$\varphi[i] = [u[i] \quad u[i-1] \quad u[i-2] \quad -y[i-1] \quad -y[i-2]]^T \qquad (7)$$

The square matrix $R_N$ is then constructed using the column vectors defined above, as shown in Eqs. 8 and 9 (step 258):

*FIR Model*

$$R_N = \sum_{i=L+1}^{N} \varphi[i]\varphi^T[i] \qquad (8)$$

*ARMA Model*

$$R_N = \sum_{i=p+1}^{N} \varphi[i]\varphi^T[i] \tag{9}$$

The column vector B is then defined from the measured output, y[i] and column vector, $\varphi$[i], as defined below in Eqs. 10 and 11 (step 260):

*FIR Model*

$$B = \sum_{i=L+1}^{N} y[i]\varphi[i] \tag{10}$$

*ARMA Model*

$$B = \sum_{i=p+1}^{N} y[i]\varphi[i] \tag{11}$$

At this point the coefficients of the FIR and ARMA models can be written as a column vector, θ, as given below in Eqs. 12 and 13:

*FIR Model*

$$\theta = [b_0 \quad b_1 \quad b_2 \quad \cdots \quad b_L]^T \tag{12}$$

*ARMA Model*

$$\theta = [b_0 \quad b_1 \quad b_2 \quad a_1 \quad a_2]^T \tag{13}$$

The square matrix and two column vectors obey the relationship given below in Eq. 14 for the adaptive filtering process.

$$R_N \theta = B \tag{14}$$

The adaptive filtering coefficients for each model can be identified from the data using the expression below in Eq. 15, where the accented column vector $\hat{\theta}$ represents the identified coefficients (step 262):

$$\hat{\theta} = R_N{}^{-1} B \tag{15}$$

Once identified, the filter coefficients can be collectively processed with the IP waveform and used to remove any motion artifacts, leaving only the respiratory component of the signal $y_R$[i], as shown below in Eq. 16 (step 264):

$$y_R[i] = y[i] - \hat{\theta}\varphi[i] \tag{16}$$

As described above, determination of the filter coefficients can be done on either the wrist-worn transceiver (Algorithm 2) or on the remote server (Algorithm 3). Once determined, the coefficients can be used to recover RR in real-time using the algorithm described above. Preferably the filter coefficients are updated when analysis of the ACC waveform indicates that the patient's motion has changed. Such a change can be represented by a change in the magnitude of the motion, a change in posture, or a change in activity state. Alternatively, the filter coefficients can be updated on a periodic basis, e.g. every 5 minutes.

There may be a time difference or delay between motion signals in the ACC waveform, and motion artifacts in the IP waveform. Such a delay, for example, may be due to real phenomena (e.g. a physiological effect) or an artifact associated with the electrical systems that measure the respective waveforms (e.g. a phase delay associated with an amplifier or analog filter that processes these waveforms). In any event, the algorithm should compensate for the delay before performing calculations shown in Eqs. 1-16, above. Such compensation can be performed using a simple time-domain analysis of ACC and IP signals influenced by a well-defined motion (e.g. a step). Alternatively, the compensation can be done during manufacturing using a one-time calibration procedure.

Figs. 15, 16, and 17 illustrate how the above-described adaptive filtering algorithm can be applied to both ACC and IP waveforms. In each of the figures, the graphs show the ACC waveform filtered with an initial, non-adaptive filter (15A, 16A, 17A; 0.01 → 2 Hz bandpass), and the IP waveform filtered under similar conditions with a slightly larger bandpass filter (15B, 16B, 17B; 0.01 → 12 Hz bandpass). Typically the IP waveform is filtered with the larger bandpass so that high-frequency components composing the rising and falling edges of pulses within these waveforms are preserved.

Once filtered, the IP waveform is processed as described above to determine an initial RR. This value may include artifacts due to motion, electrical, and mechanical noise that erroneously increases or decreases the initial RR value. But typically such errors have little impact on the final RR value that results from the adaptive filter. The middle graph (Figs. 15C, 16C, and 17C) in each figure show the IP waveform processed with the adaptive filter. In all cases this waveform features an improved signal-to-noise ratio compared to data shown in the top graph (15A, 16A, 17A), which is processed with a non-adaptive (and relatively wide) filter. Typically the narrow bandpass on the adaptive filter removes many high-frequency components that contribute the sharp rising and falling edges of pulses in the ACC waveforms. This slightly distorts the waveforms by rounding the pulses, giving the filtered waveform a shape that resembles a conventional sinusoid. Such distortion, however, has basically no affect on the absolute number of pulses in each waveform which are counted to determine RR.

The adaptively filtered IP waveform is then derivatized and graphed in Figs. 15D, 16D, and 17D. This waveform is then processed with the above-mentioned signal processing techniques, e.g. squaring the derivative and filtering out lobes that fall beneath pre-determined threshold values, to yield an algorithm-determined 'count', indicated in Figs. 15E, 16E, and 17E as a series of black triangles. The count is plotted along with the adaptively filtered IP waveforms from Figs. 15C, 16C, and 17C. Exact overlap between each pulse in the waveform and the corresponding count indicates the algorithm is working properly. Data from each of the figures correspond to varying respiratory behavior (5, 17, and 38 breaths/minute in, respectively, Figs. 15, 16, and 17), and indicate that this technique is effective over a wide range of breathing frequencies. The right-hand side of the figures (Figs. 15F, 16F, and 17F) show a series of steps 290-294 that indicate the analysis required to generate the corresponding graphs in the figure.

Algorithm 4 - Power Spectra Analysis

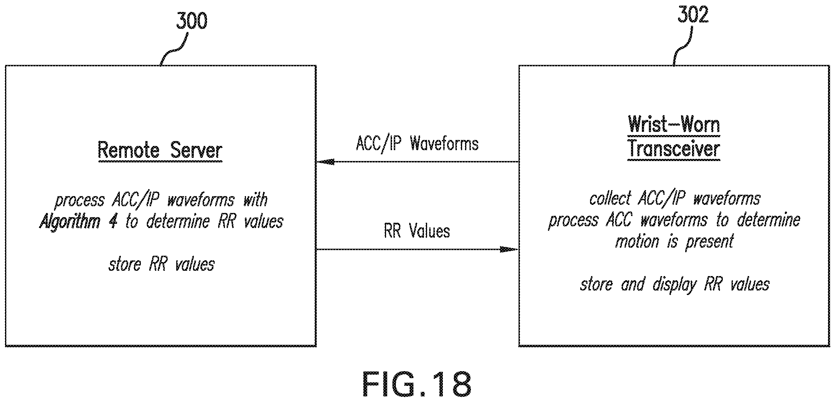

Fig. 18 shows a high-level overview of Algorithm 4, which is typically used to remove motion-related artifacts having relatively large magnitudes, such as those associated with running and walking, from the IP waveform. Algorithm 4 deconstructs both time-domain ACC and IP waveforms into their frequency-domain components, and then collectively processes these components to remove artifacts due to motion. Typically this algorithm involves collecting the two waveforms on the wrist-worn transceiver 302, and then processing them with the algorithms described above to determine if motion is present. If it is, the waveforms are wirelessly transmitted to the remote server, where they are processed with the algorithm described below to determine and then collectively process their frequency spectra to remove the affects of motion. RR is determined on the server, where it is stored, further processed, and finally sent to the wrist-worn transceiver 302 for purposed related to display and alarming.

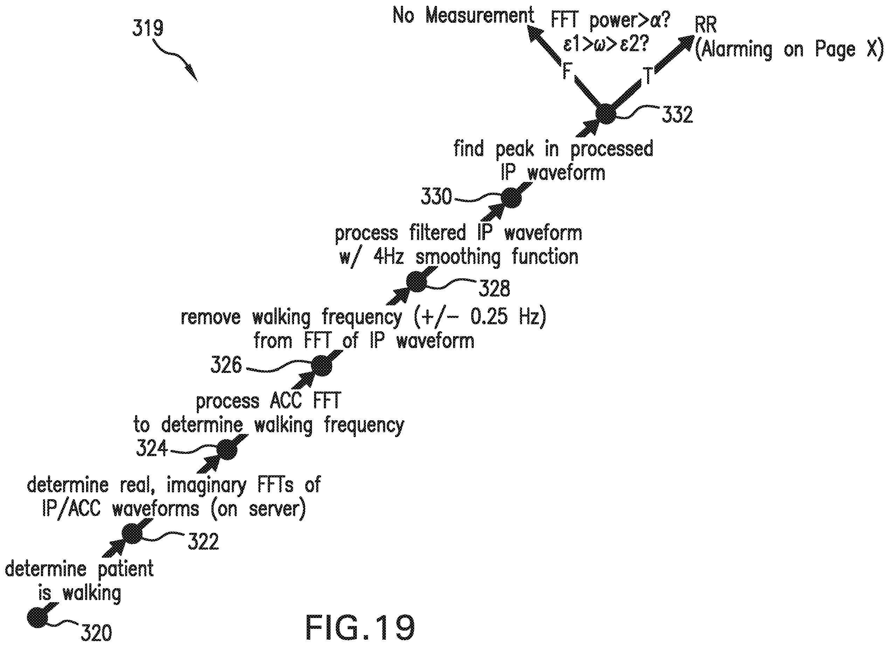

Fig. 19 shows the computational details of Algorithm 4. The algorithm begins by determining if the patient is walking or running (step 320). This is done by processing ACC waveforms according to the techniques described in the above-described patent applications. Once the patient's walking or running state is identified, the ACC and IP waveforms (represented in Eq. 17 by a(t)) are wirelessly transmitted to the remote server, which then determines their frequency-domain power spectra A(ω), as defined by Eq. 17 (step 322):

$$A(\omega) = \left[ \frac{1}{\sqrt{2\pi}} \int\limits_{-\infty}^{\infty} a(t)e^{-i\omega t} dt \right]^2 \qquad (17)$$

A(ω) shown above in Eq. 17 represents a power spectra determined from a continuous Fourier Transform. For discrete waveforms featuring an array of discrete values $a_n$, like the ones measured with the body-worn monitor, $A(\omega)$ can be rewritten as:

$$A(\omega) = \left[ \frac{1}{\sqrt{2\pi}} \sum_{n=-\infty}^{n=+\infty} a_n e^{-i\omega t} dt \right]^2 \qquad (18)$$

or alternatively as:

$$A(\omega) = \frac{F(\omega)F^*(\omega)}{2\pi} \qquad (19)$$

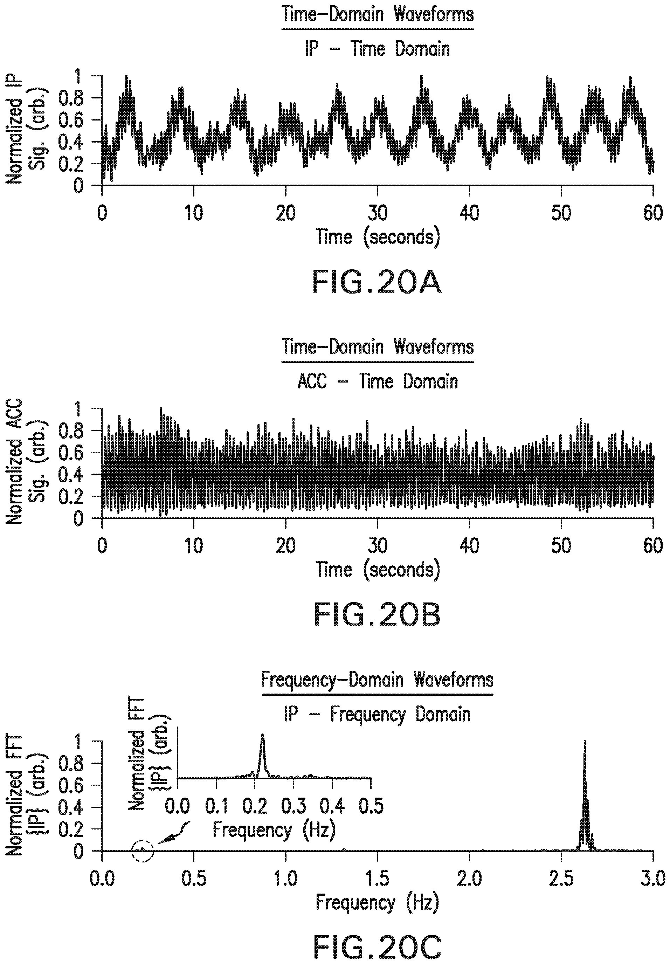

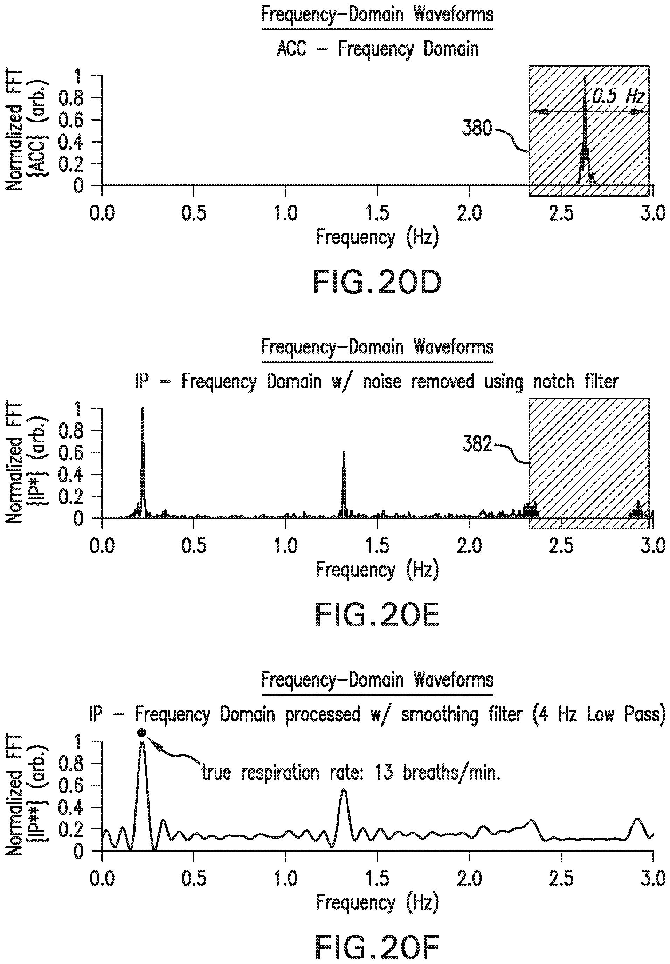

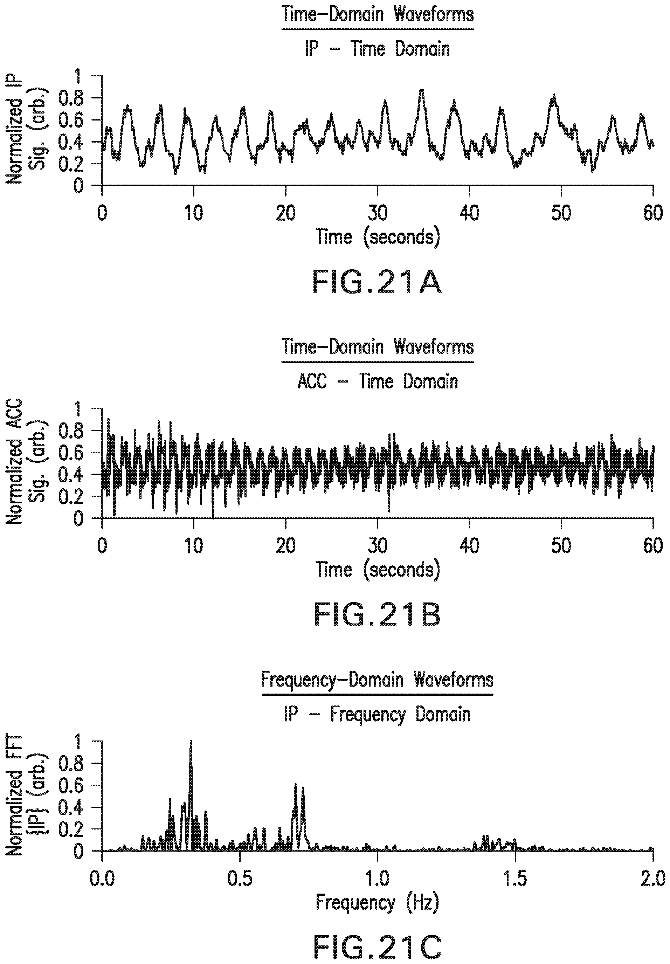

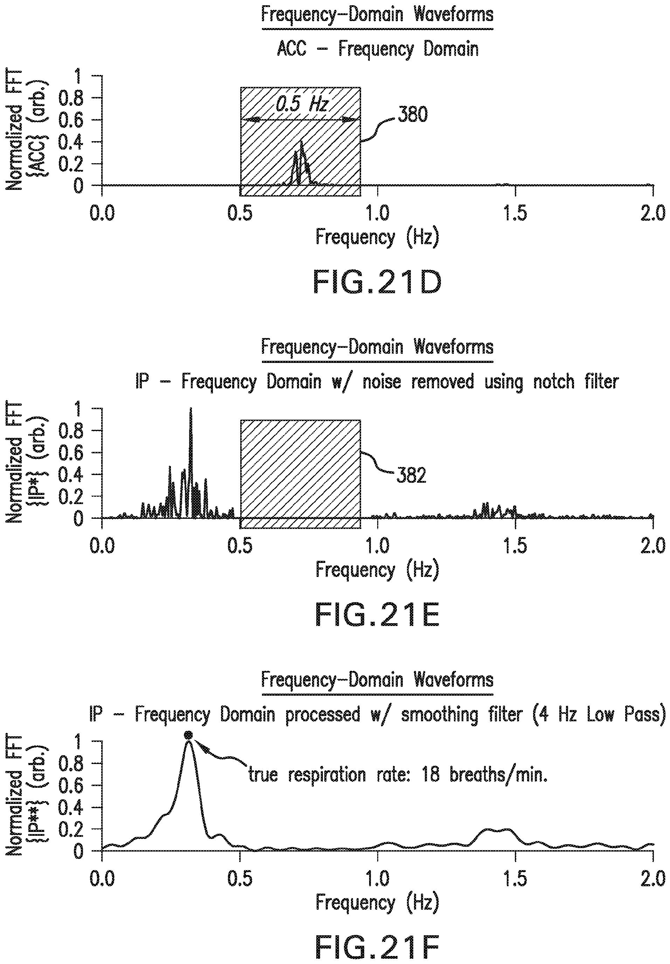

Where $F(\omega)$ is the discrete Fourier Transform and $F^*(\omega)$ is its complex conjugate. Power spectra determined this way for both IP and ACC waveforms are shown, for example, in Figs. 20C, 20D, 21C, and 21D.

The power spectra of the ACC waveform is then processed to determine a collection of frequencies corresponding to the patient's motion, which as described above corresponds to walking or running for this particular algorithm (step 324). Typically these motions are characterized by a collection of frequencies that are peaked between 0.5 and 2.0 Hz. Once the peak frequency is identified, a notch filter having a top-hat profile and a bandwidth of 0.5 Hz is constructed to surround it. Typically the primary walking frequency is positioned at the center of the notch filter, which extends 0.25 Hz on both the positive and negative ends. The notch filter is then used to process the power spectra of the IP waveform by only passing components outside of the notch (step 326). The resulting spectra of the IP waveform will now lack the frequency components related to motion. To simplify determination of the central respiratory signal, the IP waveform is then processed with a smoothing filter typically having a bandwidth of 4 Hz (step 328). Alternatively the spectrum can be processed with a rolling average. Both techniques smooth out discrete frequency components, creating a continuous set of peaks which can then be analyzed with conventional peak-finding algorithms (step 330). Examples of peak-finding algorithms include those that find the maximum frequency of each peak in the spectrum.

The frequency of the dominant peak in the filtered IP spectrum corresponds to RR. Once determined, this value and the spectrum it is extracted from can be compared to a series of pre-determined metrics to estimate the accuracy of the resulting RR (step 332). For example, the power (i.e. magnitude or density) of the peak can be compared to the metric $\alpha$ to determine if it is above a noise floor and corresponds to a level describing an actual RR. Additionally, the RR is compared to the $\varepsilon1$ and $\varepsilon2$ metrics described above to determine if the rate is within the boundaries (typically 5-60 breaths/minute) of human respiration. If so, RR is stored, further processed, and sent to the wrist-worn transceiver for display and further processing. If not, a state of 'no measurement' (indicated by dashes '---') is recorded, and the process is then repeated.

Figs. 20 and 21 show how Algorithm 4 can effectively determine RR for a patient that is running (Fig. 20) and walking (Fig. 21). Time-domain IP and ACC waveforms for the patient, collected by the body-worn monitor, are shown respectively in Figs. 20A and 20B. In the IP waveform, the slowly varying pulses occurring approximately every 5 seconds correspond to individual breaths, while the sharp peaks in both the ACC and IP waveforms that occur roughly twice each second corresponds to running steps. In this case, motion artifacts from the running motion clearly couple into the IP waveform.

Figs. 20C and 20D show, respectively, frequency-domain power spectra of both the IP and ACC waveforms. Clearly shown in the power spectra of the IP waveform is a dominant peak near 2.7 Hz corresponding to motion artifacts from the patient's running. A much weaker peak is also evident near 0.2 Hz. The power spectra corresponding to the ACC waveform features only a peak near 2.7 Hz that includes nearly the exact same frequency components as the corresponding peak in the IP waveform. As shown in Fig. 20D, a top-hat shaped notch filter centered at 2.7 Hz with a bandwidth of 0.5 Hz, indicated by the gray area 380, is then applied to the IP waveform. This yields the filtered waveform, shown in Fig. 20E, that lacks the high-frequency peak associated with the motion artifacts, as shown by the gray area 382. The relatively low-frequency peak near 0.2 Hz is now dominant. Further processing of this spectrum with a 4 Hz smoothing function eliminates most of the jagged edges present in Fig. 20E, yielding the continuous power spectrum shown in Fig. 20F. Processing this spectrum with a simple peak-finding algorithm yields the patient's actual RR, which corresponds to about 13 breaths/minute.

Fig. 21 shows a case where the RR and the motion artifact, in this case caused by walking, are relatively close in frequency. Here, the time-domain IP waveform shown in Fig. 21A lacks a strong, periodic respiratory signal, and is strongly corrupted by the walking-induced signals in the ACC waveform, shown in Fig. 21B. Each pulse in the ACC waveform corresponds to a unique step. The corresponding power spectra, shown in Figs. 21C and 21D, show frequency components corresponding to both the motion artifact (near 0.75 Hz) and the respiratory signal (near 0.25 Hz). Because the respiratory signal lacks a clear, well-defined breathing pattern, the corresponding power spectrum features a range a frequencies between 0.2 and 0.5 Hz.

Application of the 0.5 Hz bandwidth notch filter, shown by the gray area 380 in Fig. 21D, removes motion artifacts from the IP waveform, leaving only a range of frequencies between 0.2 and 0.5 Hz. This portion of the spectrum features a large number of sharp peaks which are difficult to isolate with conventional peak-finding algorithms. However application of the 4 Hz smoothing function, as indicated by Fig. 21D, reduces this collection of frequencies to a single, broad peak shown in Fig. 21E that corresponds to the patient breathing at about 18 breaths/minute.

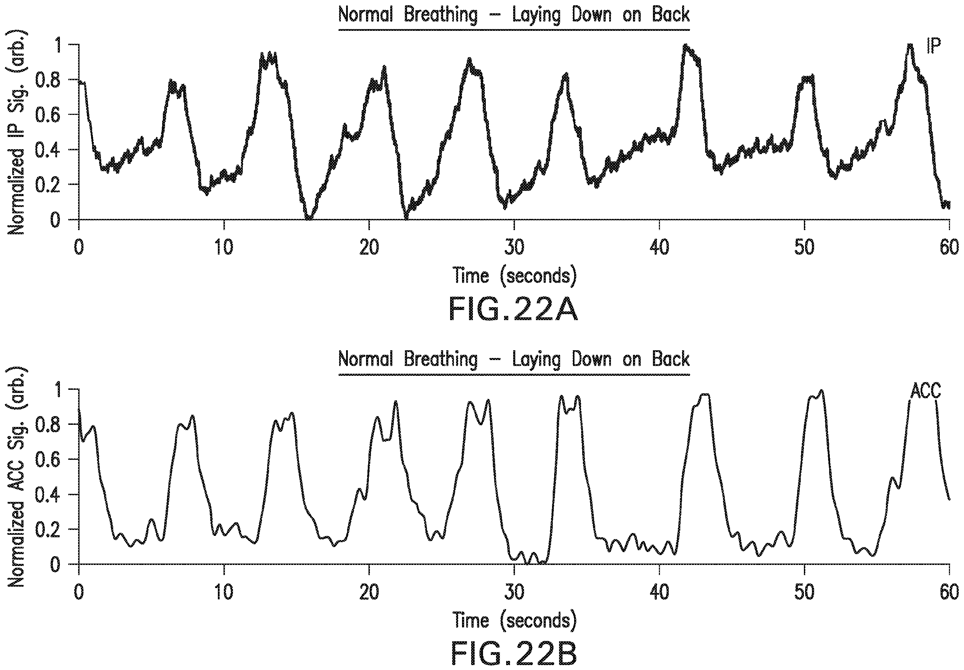

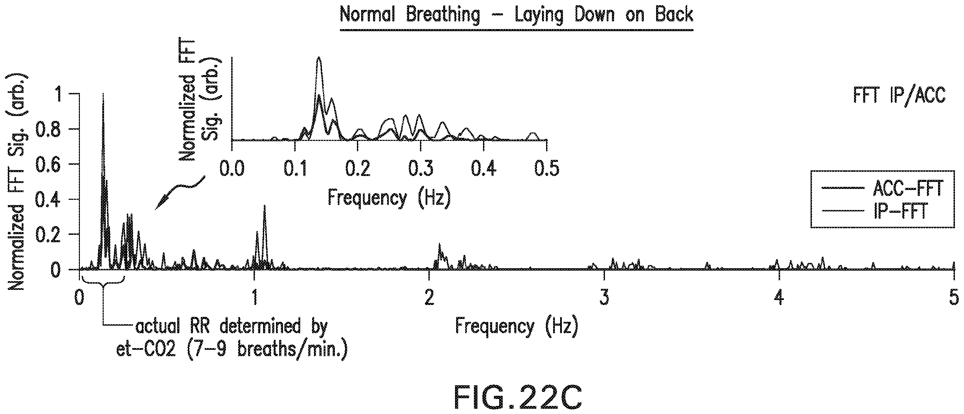

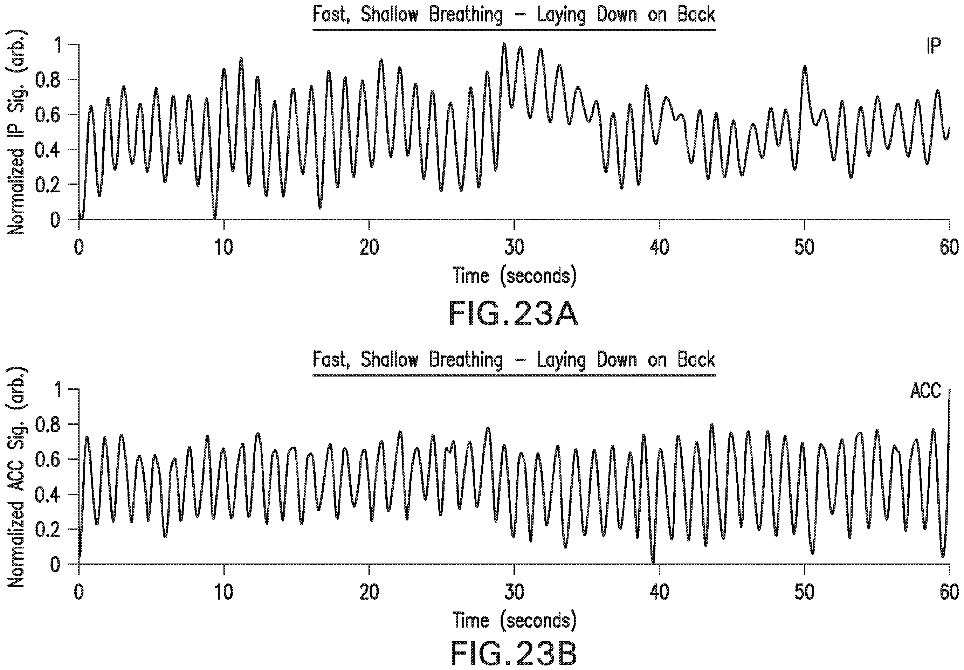

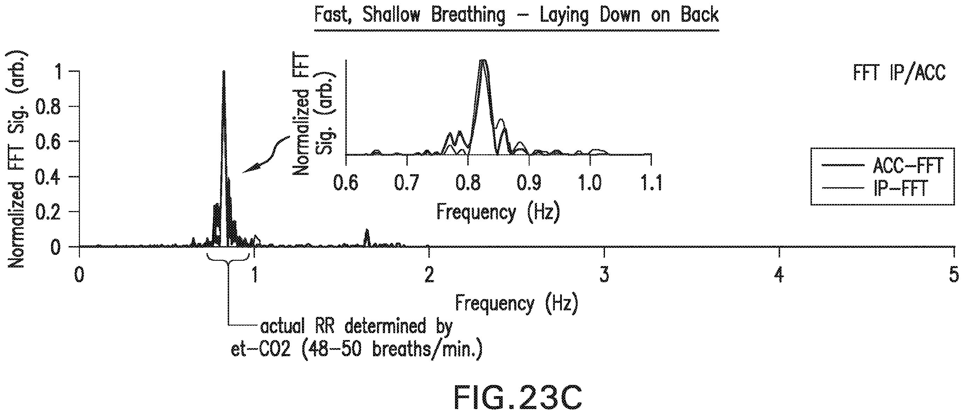

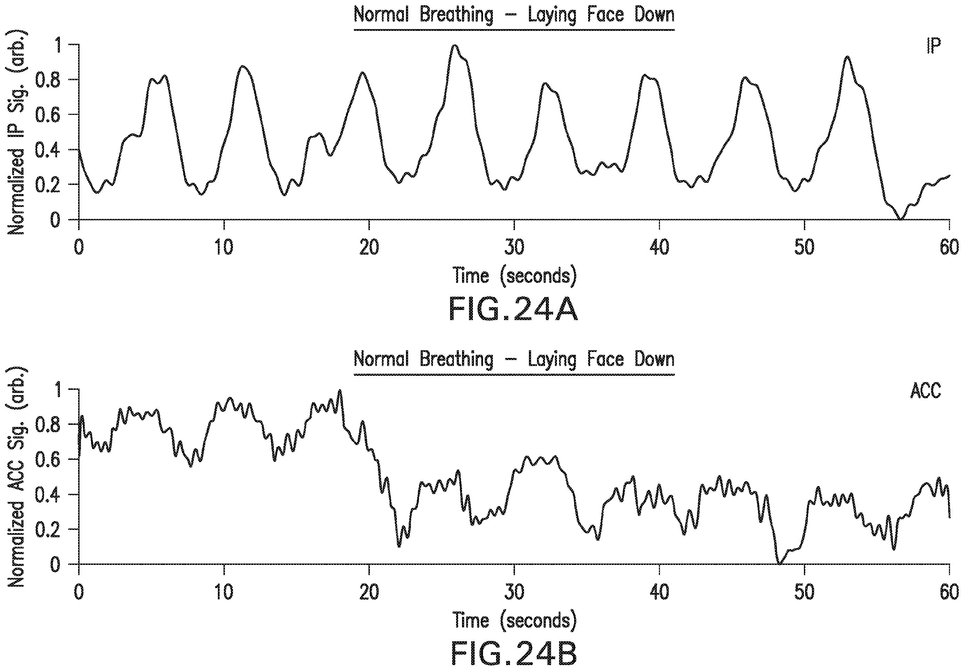

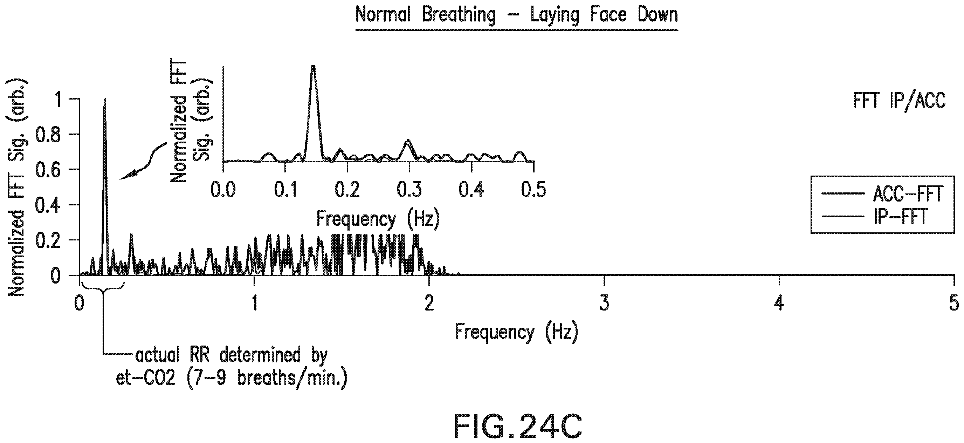

As shown in Figs. 22-24, when the patient is stationary, ACC waveforms measured from the patient's chest (or, alternatively, belly) are sensitive to RR. This is because slight, breathing-induced motions of the patient's torso can be detected by the accelerometer. These figures show the time-domain IP waveforms (Figs. 22A, 23A, 24A) and ACC waveforms (Figs. 22B, 23B, 24B), both of which show pulses, lined up exactly in time, that correspond to individual breaths. The frequency-domain power spectrum (Figs. 22C, 23C, 24C) show how the frequency components of these time-domain waveform are roughly equivalent, indicating both waveforms are sensitive to RR. This equivalency holds for normal breathing when the patient is on their back (Fig. 22); fast, shallow breathing when the patient is on their back (Fig. 23); and normal breathing when the patient is lying face down (Fig. 24). In all these cases both the ACC and IP waveforms can be processed collectively or independently to estimate RR. For collective processing, RRs may be determined from both waveforms, and then averaged together to generate a single value. Or a value may be determined from each waveform, and then subjected to a series of metrics (like those described above using $\alpha$, $\beta$, $\varepsilon1$, $\varepsilon2$) to determine if one or both of the calculated RRs is valid. Processing of the waveforms may involve simple pulse-counting algorithms that analyze time-domain waveforms, like the ones shown in Figs. 22A, 22B, 23A, 23B, 24A, 24B, or more sophisticated spectral-analysis algorithms that analyze frequency-domain waveforms, like the ones shown in Figs. 22C, 23C, 24C.

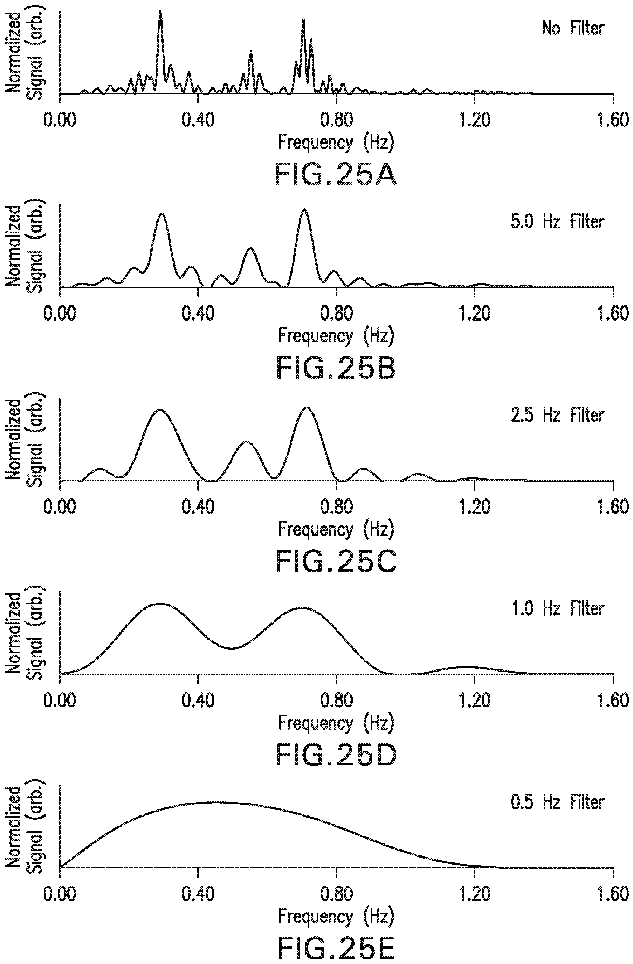

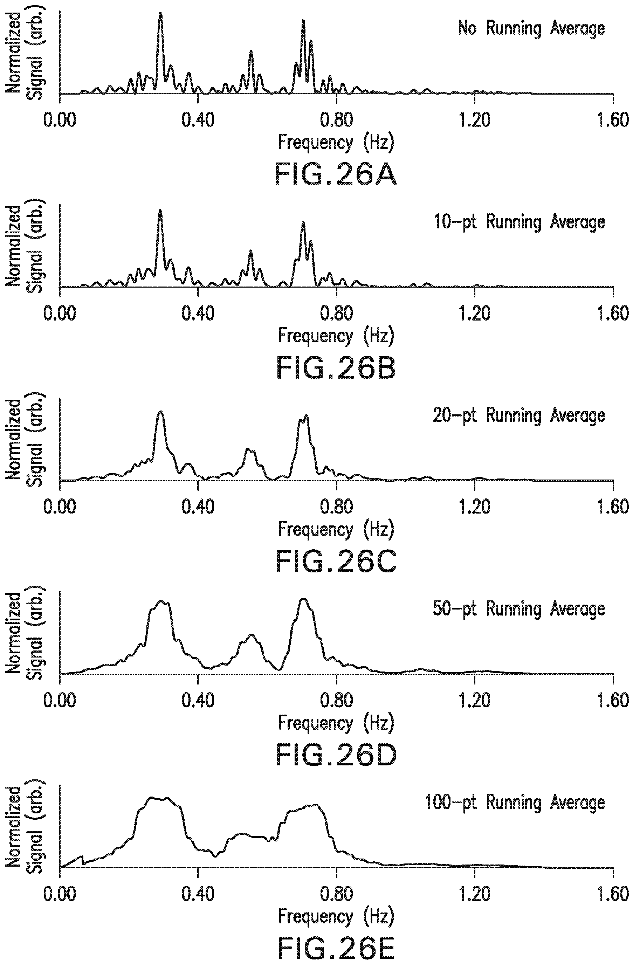

Figs. 25 and 26 show how a filtering algorithm (Fig. 25) and rolling average (Fig. 26), when applied to a conventional power spectrum, facilitates determination of RR. The power spectrum in Fig. 25A (shown in the figure's top portion) is unprocessed, and features a collection of peaks positioned in three groupings near 0.3, 0.6, and 0.7 Hz. As described above, a low-pass smoothing filter based on a FIR function smoothes out these sharply varying features, resulting in a continuous spectrum that is relatively easy to analyze to determine a single frequency corresponding to RR. In this case, the smoothing filter is processing the power spectrum as if it were a time-domain waveform. Fig. 25B, for example, shows the spectrum after processing with a 5 Hz low-pass filter. Here, the individual peaks are mostly smoothed out, resulting in three primary peaks related to RR. Note that these peaks are not necessarily artifacts, and instead are due to physiological variations in the patient's breathing pattern. Progressively decreasing the filter cutoff removes more and more of the features that result in sharply varying peaks in the frequency spectrum. Ultimately when a 0.5 Hz filter is used, this results in a single, broad peak that lacks the definition to accurately determine RR. As described with reference to Figs. 20F and 21F, the ideal smoothing filter has a cutoff of about 4 Hz.

Fig. 26 shows an alternate embodiment of the invention wherein a rolling average is used in place of the smoothing filter described above with reference to Fig. 25. Here, the rolling average is gradually increased from 0 points (Fig. 26A, at the top of the page) to 100 points. When the average is increased above about 20 points the features in the frequency spectrum begin to blend together to form a well-defined peak that can be easily analyzed using the peak-finding algorithm referenced above.

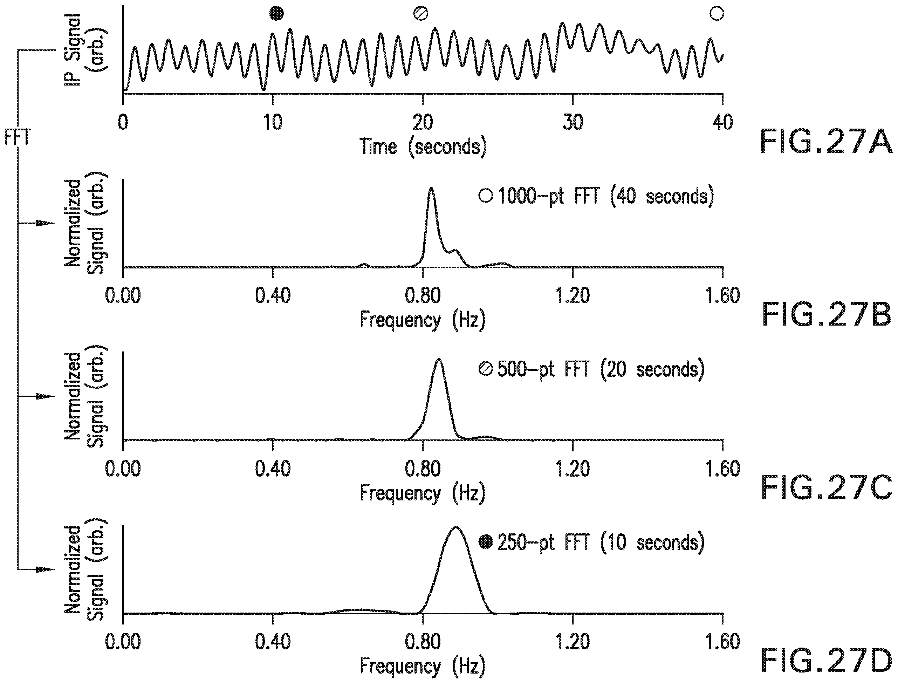

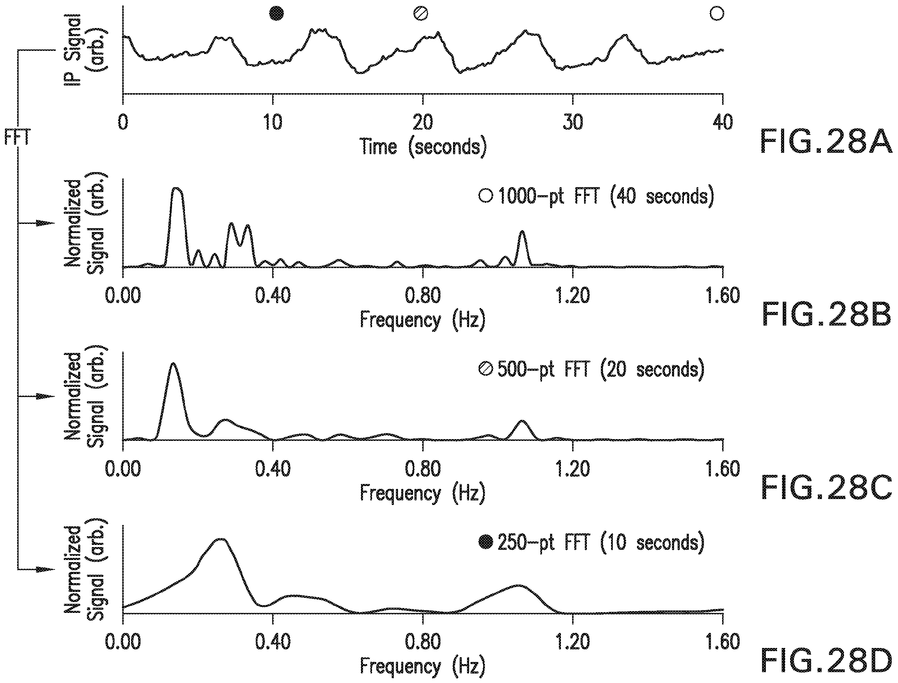

Referring to Figs. 27 and 28, the number of time-domain samples used to calculate an associated Fourier Transform and power spectrum will have a significant influence on the resolution of the power spectrum. In general, increasing the number of time-domain samples will increase resolution in the power spectrum. According to this invention, the frequency-domain resolution can be arbitrarily increased by adding constant values to the time-domain ACC and IP waveform. In this technique, typically called 'zero padding', the constant values are typically 0, or alternatively a constant value.

Both Fig. 27 and 28 show a time-domain IP waveform (Figs. 27A and 28A, shown at the top of the page) along with associated power spectra calculated, respectively, using 1000 points (Figs. 27B, 28B), 500 points (Fig. 27C, 28C), and 250 points (Fig. 27D, 28D). The IP waveform for this figure was sampled at 25 Hz, and thus the time-domain markers that correspond to the above-mentioned points are indicated by a light gray circle (1000 points), a dark gray circle (500 points), and a black circle (250 points). As shown in Figs. 27B, 28B, 27C, 28C, power spectra determined with both 1000-point and 500-point Fourier Transforms feature roughly the same frequency profile, with, as expected, the 1000-point spectra showing relatively greater resolution. Power spectra calculated from the 250-point Fourier Transform show one or more very broad peaks, and, importantly, undergo a frequency shift which trends toward higher frequencies for the dominant peak. Such a frequency shift will result in an erroneous value for the eventual RR. For this reason, it is preferred that the Fourier Transform calculation use a minimum of 500 points, which corresponds to 10 seconds of data sampled at 50 Hz for the time-domain ACC and IP waveforms. The ideal number of points for this calculation is about 750, which at 50 Hz can be achieved in 15 seconds. Additionally, the frequency-domain data shown in Figs. 27 and 28 was calculated with substantially fewer time-domain samples than that used for all previous figures (e.g. Figs. 20, 21; Fourier Transforms for these figures were calculated with approximately 5000 points). This results in a relatively low-resolution power spectrum that may not need the additional 4 Hz smoothing filter, indicated above with reference to Figs. 20F and 21F. Here, the smoothing filter is essentially artificially applied due to the relatively low resolution of the power spectra.

Processing ACC Waveforms to Determine Posture

In addition to activity level, as described above and indicated in Figs. 21-24, a patient's posture can influence how the above-described system generates alarms/alerts from RR, cNIBP, and other vital signs. For example, the alarms/alerts related to both RR and cNIBP may vary depending on whether the patient is lying down or standing up. Fig. 29 indicates how the body-worn monitor can determine motion-related parameters (e.g. degree of motion, posture, and activity level) from a patient 410 using time-dependent ACC waveforms continuously generated from the three accelerometers 412, 413, 414 worn, respectively, on the patient's chest, bicep, and wrist. The height of the patient's arm can affect the cNIBP measurement, as blood pressure can vary significantly due to hydrostatic forces induced by changes in arm height. Moreover, this phenomenon can be detected and exploited to calibrate the cNIBP measurement, as described in detail in the above-referenced patent applications. As described in these documents, arm height can be determined using DC signals from the accelerometers 413, 414 disposed, respectively, on the patient's bicep and wrist. Posture, in contrast, can be exclusively determined by the accelerometer 412 worn on the patient's chest. An algorithm operating on the wrist-worn transceiver extracts DC values from waveforms measured from this accelerometer and processes them with an algorithm described below to determine posture.

Specifically, torso posture is determined for a patient 410 using angles determined between the measured gravitational vector and the axes of a torso coordinate space 411. The axes of this space 411 are defined in a three-dimensional Euclidean space where $\vec{R}_{CV}$ is the vertical axis, $\vec{R}_{CH}$ is the horizontal axis, and $\vec{R}_{CN}$ is the normal axis. These axes must be identified relative to a 'chest accelerometer coordinate space' before the patient's posture can be determined.

The first step in determining a patient's posture is to identify alignment of $\vec{R}_{CV}$ in the chest accelerometer coordinate space. This can be determined in either of two approaches. In the first approach, $\vec{R}_{CV}$ is assumed based on a typical alignment of the body-worn monitor relative to the patient. During a manufacturing process, these parameters are then preprogrammed into firmware operating on the wrist-worn transceiver. In this procedure it is assumed that accelerometers within the body-worn monitor are applied to each patient with essentially the same configuration. In the second approach, $\vec{R}_{CV}$ is identified on a patient-specific basis. Here, an algorithm operating on the wrist-worn transceiver prompts the patient (using, e.g., video instruction operating on the wrist-worn transceiver, or audio instructions transmitted through a speaker) to assume a known position with respect to gravity (e.g., standing upright with arms pointed straight down). The algorithm then calculates $\vec{R}_{CV}$ from DC values corresponding to the x, y, and z axes of the chest accelerometer while the patient is in this position. This case, however, still requires knowledge of which arm (left or right) the monitor is worn on, as the chest accelerometer coordinate space can be rotated by 180 degrees depending on this orientation. A medical professional applying the monitor can enter this information using the GUI, described above. This potential for dual-arm attachment requires a set of two pre-determined vertical and normal vectors which are interchangeable depending on the monitor's location. Instead of manually entering this information, the arm on which the monitor is worn can be easily determined following attachment using measured values from the chest accelerometer values, with the assumption that $\vec{R}_{CV}$ is not orthogonal to the gravity vector.

The second step in the procedure is to identify the alignment of $\vec{R}_{CN}$ in the chest accelerometer coordinate space. The monitor determines this vector in the same way it determines $\vec{R}_{CV}$ using one of two approaches. In the first approach the monitor assumes a typical alignment of the chest-worn accelerometer on the patient. In the second approach, the alignment is identified by prompting the patient to assume a known position with respect to gravity. The monitor then calculates $\vec{R}_{CN}$ from the DC values of the time-dependent ACC waveform.

The third step in the procedure is to identify the alignment of $\vec{R}_{CH}$ in the chest accelerometer coordinate space. This vector is typically determined from the vector cross product of $\vec{R}_{CV}$ and $\vec{R}_{CN}$, or it can be assumed based on the typical alignment of the accelerometer on the patient, as described above.

A patient's posture is determined using the coordinate system described above and in Fig. 29, along with a gravitational vector $\vec{R}_{G}$ that extends normal from the patient's chest. The angle between $\vec{R}_{CV}$ and $\vec{R}_{G}$ is given by Eq. 20:

$$\theta_{VG}[n] = arccos\left(\frac{\vec{R}_G[n] \cdot \vec{R}_{CV}}{\left\|\vec{R}_G[n]\right\|\left\|\vec{R}_{CV}\right\|}\right) \tag{20}$$

where the dot product of the two vectors is defined as:

$$\vec{R}_G[n] \cdot \vec{R}_{CV} = \left(y_{Cx}[n] \times r_{CVx}\right) + \left(y_{Cy}[n] \times r_{CVy}\right) + \left(y_{Cz}[n] \times r_{CVz}\right) \tag{21}$$

The definition of the norms of $\vec{R}_G$ and $\vec{R}_{CV}$ are given by Eqs. 22 and 23:

$$\left\|\vec{R}_G[n]\right\| = \sqrt{(y_{Cx}[n])^2 + \left(y_{Cy}[n]\right)^2 + (y_{Cz}[n])^2} \tag{22}$$

$$\left\|\vec{R}_{CV}\right\| = \sqrt{(r_{CVx})^2 + \left(r_{CVy}\right)^2 + (r_{CVz})^2} \tag{23}$$

As indicated in Eq. 24, the monitor compares the vertical angle $\theta_{VG}$ to a threshold angle to determine whether the patient is vertical (i.e. standing upright) or lying down:

$$if\ \theta_{VG} \le 45°\ then\ Torso\ State = 0, the\ patient\ is\ upright \tag{24}$$

If the condition in Eq. 24 is met the patient is assumed to be upright, and their torso state, which is a numerical value equated to the patient's posture, is equal to 0. The patient is assumed to be lying down if the condition in equation (5) is not met, i.e. $\theta_{VG} > 45$ degrees. Their lying position is then determined from angles separating the two remaining vectors, as defined below.

The angle $\theta_{NG}$ between $\vec{R}_{CN}$ and $\vec{R}_G$ determines if the patient is lying in the supine position (chest up), prone position (chest down), or on their side. Based on either an assumed orientation or a patient-specific calibration procedure, as described above, the alignment of $\vec{R}_{CN}$ is given by Eq. 25, where *i, j, k* represent the unit vectors of the x, y, and z axes of the chest accelerometer coordinate space respectively:

$$\vec{R}_{CN} = r_{CNx}\hat{\imath} + r_{CNy}\hat{\jmath} + r_{CNz}\hat{k} \tag{25}$$

The angle between $\vec{R}_{CN}$ and $\vec{R}_G$ determined from DC values extracted from the chest ACC waveform is given by Eq. 26:

$$\theta_{NG}[n] = arccos\left(\frac{\vec{R}_G[n] \cdot \vec{R}_{CN}}{\left\|\vec{R}_G[n]\right\|\left\|\vec{R}_{CN}\right\|}\right) \tag{26}$$

The body-worn monitor determines the normal angle $\theta_{NG}$ and then compares it to a set of predetermined threshold angles to determine which position in which the patient is lying, as shown in Eq. 27:

$$if\ \theta_{NG} \le 35°\ then\ Torso\ State = 1, the\ patient\ is\ supine$$

$$if\ \theta_{NG} \ge 135°\ then\ Torso\ State = 2, the\ patient\ is\ prone \tag{27}$$

If the conditions in Eq. 27 are not met then the patient is assumed to be lying on their side. Whether they are lying on their right or left side is determined from the angle calculated between the horizontal torso vector and measured gravitational vectors, as described above.

The alignment of $\vec{R}_{CH}$ is determined using either an assumed orientation, or from the vector cross-product of $\vec{R}_{CV}$ and $\vec{R}_{CN}$ as given by Eq. 28, where *i, j, k* represent the unit vectors of the x, y, and z axes of the accelerometer coordinate space respectively. Note that the orientation of the calculated vector is dependent on the order of the vectors in the operation. The order below defines the horizontal axis as positive towards the right side of the patient's body.

$$\vec{R}_{CH} = r_{CVx}\hat{\imath} + r_{CVy}\hat{\jmath} + r_{CVz}\hat{k} = \vec{R}_{CV} \times \vec{R}_{CN} \qquad (28)$$

The angle $\theta_{HG}$ between $\vec{R}_{CH}$ and $\vec{R}_G$ is determined using Eq. 29:

$$\theta_{HG}[n] = arccos\left(\frac{\vec{R}_G[n] \cdot \vec{R}_{CH}}{\left\|\vec{R}_G[n]\right\|\left\|\vec{R}_{CH}\right\|}\right) \qquad (29)$$

The monitor compares this angle to a set of predetermined threshold angles to determine if the patient is lying on their right or left side, as given by Eq. 30:

$$if\ \theta_{HG} \geq 90°\ then\ Torso\ State = 3, the\ patient\ is\ on\ their\ right\ side$$

$$(30)$$

$$if\ \theta_{NG} < 90°\ then\ Torso\ State = 4, the\ patient\ is\ on\ their\ left\ side$$

Table 1 describes each of the above-described postures, along with a corresponding numerical torso state used to render, e.g., a particular icon on a remote computer:

**Table 1 - postures and their corresponding torso states**

| Posture | Torso State |
|---|---|
| standing upright | 0 |
| supine: lying on back | 1 |
| prone: lying on chest | 2 |
| lying on right side | 3 |
| lying on left side | 4 |
| undetermined posture | 5 |

Figs. 30A and 30B show, respectively, graphs of time-dependent ACC waveforms measured along the x, y, and z-axes (Fig. 30A), and the torso states (i.e. postures; Fig. 30B) determined from these waveforms for a moving patient, as described above. As the patient moves, the DC values of the ACC waveforms measured by the chest accelerometer vary accordingly, as shown in Fig. 30A. The body-worn monitor processes these values as described above to continually determine $\vec{R}_G$ and the various quantized torso states for the patient, as shown in Fig. 30B. The torso states yield the patient's posture as defined in Table 1. For this study the patient rapidly alternated between standing, lying on their back, chest, right side, and left side within a time period of about 160 seconds. As described above, different alarm/alert conditions (e.g. threshold values) for vital signs can be assigned to each of these postures, or the specific posture itself may result in an alarm/alert. Additionally, the time-dependent properties of the graph can be analyzed (e.g. changes in the torso states can be counted) to determine, for example, how often the patient moves in their hospital bed. This number can then be equated to various metrics, such as a 'bed sore index' indicating a patient that is so stationary in their bed that lesions may result. Such a state could then be used to trigger an alarm/alert to the supervising medical professional.

Hardware for Measuring RR

Figs. 4A and 4B show how the body-worn monitor 100 described above attaches to a patient 70 to measure RR, cNIBP, and other vital signs. These figures show two configurations of the system: Fig. 4A shows the system used during the indexing portion of the Composite Technique, and includes a pneumatic, cuff-based system 85, while Fig. 4B shows the system used for subsequent RR and cNIBP measurements. The indexing measurement typically takes about 60 seconds, and is typically performed once every 4 hours. Once the indexing measurement is complete the cuff-based system 85 is typically removed from the patient. The remainder of the time the monitor 100 performs the RR, HR, SpO2 and cNIBP measurements.

The body-worn monitor 100 features a wrist-worn transceiver 72, described in more detail in Fig. 5, featuring a touch panel interface 73 that displays RR and other vital signs. A wrist strap 90 affixes the transceiver 72 to the patient's wrist like a conventional wristwatch. A flexible cable 92 connects the transceiver 72 to an optical sensor 94 that wraps around the base of the patient's thumb. During a measurement, the optical sensor 94 generates a time-dependent PPG waveform which is processed along with an ECG to measure cNIBP, SpO2, and, in some applications, RR. To determine ACC waveforms the body-worn monitor 100 features three separate accelerometers located at different portions on the patient's arm and chest. The first accelerometer is surface-mounted on a circuit board in the wrist-worn transceiver 72 and measures signals associated with movement of the patient's wrist. As described above, this motion can also be indicative of that originating from the patient's fingers, which will affect the SpO2 measurement. The second accelerometer is included in a small bulkhead portion 96 included along the span of the cable 82. During a measurement, a small piece of disposable tape, similar in size to a conventional bandaid, affixes the bulkhead portion 96 to the patient's arm. In this way the bulkhead portion 96 serves two purposes: 1) it measures a time-dependent ACC waveform from the mid-portion of the patient's arm, thereby allowing their posture and arm height to be determined as described in detail above; and 2) it secures the cable 82 to the patient's arm to increase comfort and performance of the body-worn monitor 100, particularly when the patient is ambulatory. The third accelerometer is mounted in the sensor module 74 that connects through cables 80a-c to ECG electrodes 78a-c. As described in detail above, this accelerometer, which can also be mounted closer to the patient's belly, measures respiration-induced motion of the patient's chest and belly. These signals are then digitized, transmitted through the cable 82 to the wrist-worn transceiver 72, where they are processed with an algorithm as described above to determine RR.

The cuff-based module 85 features a pneumatic system 76 that includes a pump, valve, pressure fittings, pressure sensor, manifold, analog-to-digital converter, microcontroller, and rechargeable Li:ion battery. During an indexing measurement, the pneumatic system 76 inflates a disposable cuff 84 and performs two measurements according to the Composite Technique: 1) it performs an inflation-based measurement of oscillometry to determine values for SYS, DIA, and MAP; and 2) it determines a patient-specific relationship between PTT and MAP. These measurements are described in detail in the above-referenced patent applications entitled: 'VITAL SIGN MONITOR FOR MEASURING BLOOD PRESSURE USING OPTICAL, ELECTRICAL, AND PRESSURE WAVEFORMS' (U.S.S.N 12/138,194; filed June 12, 2008) and 'BODY-WORN SYSTEM FOR MEASURING CONTINUOUS NON-INVASIVE BLOOD PRESSURE (cNIBP)' (U.S.S.N 12/650,354; filed November 15, 2009).

The cuff 84 within the cuff-based pneumatic system 85 is typically disposable and features an internal, airtight bladder that wraps around the patient's bicep to deliver a uniform pressure field. During the indexing measurement, pressure values are digitized by the internal analog-to-digital converter, and sent through a cable 86 according to a CAN protocol, along with SYS, DIA, and MAP blood pressures, to the wrist-worn transceiver 72 for processing as described above. Once the cuff-based measurement is complete, the cuff-based module 85 is removed from the patient's arm and the cable 86 is disconnected from the wrist-worn transceiver 72. cNIBP is then determined using PTT, as described in detail above.

To determine an ECG, the body-worn monitor 100 features a small-scale, three-lead ECG circuit integrated directly into the sensor module 74 that terminates an ECG cable 82. The ECG circuit features an integrated circuit that collects electrical signals from three chest-worn ECG electrodes 78a-c connected through cables 80a-c. As described above, the ECG electrodes 78a-c are typically disposed in a conventional Einthoven's Triangle configuration, which is a triangle-like orientation of the electrodes 78a-c on the patient's chest that features three unique ECG vectors. From these electrical signals the ECG circuit determines up to three ECG waveforms, which are digitized using an analog-to-digital converter mounted proximal to the ECG circuit, and sent through the cable 82 to the wrist-worn transceiver 72 according to the CAN protocol. There, the ECG and PPG waveforms are processed to determine the patient's blood pressure. Heart rate and RR are determined directly from the ECG waveform using known algorithms, such as those described above. More sophisticated ECG circuits (e.g. five and twelve-lead systems) can plug into the wrist-worn transceiver to replace the three-lead system shown in Figs. 4A and 4B.

Fig. 5 shows a close-up view of the wrist-worn transceiver 72. As described above, it attaches to the patient's wrist using a flexible strap 90 which threads through two D-ring openings in a plastic housing 106. The transceiver 72 features a touchpanel display 120 that renders a GUI 73 which is altered depending on the viewer (typically the patient or a medical professional). Specifically, the transceiver 72 includes a small-scale infrared barcode scanner 102 that, during use, can scan a barcode worn on a badge of a medical professional. The barcode indicates to the transceiver's software that, for example, a nurse or doctor is viewing the user interface. In response, the GUI 73 displays vital sign data and other medical diagnostic information appropriate for medical professionals. Using this GUI 73, the nurse or doctor, for example, can view the vital sign information, set alarm parameters, and enter information about the patient (e.g. their demographic information, medication, or medical condition). The nurse can press a button on the GUI 73 indicating that these operations are complete. At this point, the display 120 renders an interface that is more appropriate to the patient, such as time of day and battery power.

The transceiver 72 features three CAN connectors 104a-c on the side of its upper portion, each which supports the CAN protocol and wiring schematics, and relays digitized data to the internal CPU. Digital signals that pass through the CAN connectors include a header that indicates the specific signal (e.g. ECG, ACC, or pressure waveform from the cuff-based module) and the sensor from which the signal originated. This allows the CPU to easily interpret signals that arrive through the CAN connectors 104a-c, such as those described above corresponding to RR, and means that these connectors are not associated with a specific cable. Any cable connecting to the transceiver can be plugged into any connector 104a-c. As shown in Fig. 4A, the first connector 104a receives the cable 82 that transports a digitized ECG waveform determined from the ECG circuit and electrodes, and digitized ACC waveforms measured by accelerometers in the sensor module 74 and the bulkhead portion 96 associated with the ECG cable 82.

The second CAN connector 104b shown in Fig. 5 receives the cable 86 that connects to the pneumatic cuff-based system 85 used for the pressure-dependent indexing measurement (shown in Fig. 4A). This connector 104b receives a time-dependent pressure waveform delivered by the pneumatic system 85 to the patient's arm, along with values for SYS, DIA, and MAP values determined during the indexing measurement. The cable 86 unplugs from the connector 104b once the indexing measurement is complete, and is plugged back in after approximately four hours for another indexing measurement.

The final CAN connector 104c can be used for an ancillary device, e.g. a glucometer, infusion pump, body-worn insulin pump, ventilator, or et-CO2 measurement system. As described above, digital information generated by these systems will include a header that indicates their origin so that the CPU can process them accordingly.

The transceiver includes a speaker 101 that allows a medical professional to communicate with the patient using a voice over Internet protocol (VOIP). For example, using the speaker 101 the medical professional could query the patient from a central nursing station or mobile phone connected to a wireless, Internet-based network within the hospital. Or the medical professional could wear a separate transceiver similar to the shown in Fig. 5, and use this as a communication device. In this application, the transceiver 72 worn by the patient functions much like a conventional cellular telephone or 'walkie talkie': it can be used for voice communications with the medical professional and can additionally relay information describing the patient's vital signs and motion. The speaker can also enunciate pre-programmed messages to the patient, such as those used to calibrate the chest-worn accelerometers for a posture calculation, as described above.

Other embodiments which differ from the Invention

RR can also be calculated using a combination of ACC, ECG, PPG, IP, and other signals using algorithms that differ from those described above. For example, these signals can be processed with an averaging algorithm, such as one using a weighted average, to determine a single waveform that can then be processed to determine RR. Some of these measurement techniques are described, for example, in the following patent applications: 'BODY-WORN MON-ITOR FOR MEASURING RESPIRATION RATE' (U.S.S.N 12/559,419; filed September 14, 2009). Or the ACC waveform can be used alone, without being integrated in an adaptive filtering algorithm, to determine RR without relying on IP. In this case the ACC waveform is filtered with a simple bandpass filter, e.g. a finite impulse response filter, with a set passband (e.g. 0.01 → 5 Hz). Similarly, multiple ACC waveforms, such as those measured along axes (e.g. the x or y-axes) orthogonal to the vector normal to the patient's chest (i.e. the z-axis), can be processed with or without adaptive filtering to determine RR. In this case the waveforms may be averaged together with a weighted average to generate a single waveform, which is then filtered, derivatized, and signal processed as described above to determine RR. Similarly, envelopes associated with the ECG and PPG waveforms can be processed in a similar manner to determine RR. In still other embodiments, other sensors, such as detectors for ultra wide-band radar or acoustic microphones, can detect signals indicative of RR and used with ACC or IP waveforms and the adaptive filtering approach described above to determine RR. Here, the alternative sensors are typically used to replace measurement of the IP waveform, although they can also be used to replace measurement of the ACC waveform. An acoustic sensor suitable for this application is described, for example, in the following co-pending applications: DEVICE FOR DETERMINING RESPIRATORY RATE AND OTHER VITAL SIGNS (U.S.S.N. 12/171,886; filed July 12, 2008).

In addition to those methods described above, the body-worn monitor can use a number of additional methods to calculate blood pressure and other properties from the optical and electrical waveforms. These are described in the following co-pending patent applications: 1) CUFFLESS BLOOD-PRESSURE MONITOR AND ACCOMPANYING WIRELESS, INTERNET-BASED SYSTEM (U.S.S.N 10/709,015; filed April 7, 2004); 2) CUFFLESS SYSTEM FOR MEASURING BLOOD PRESSURE (U.S.S.N. 10/709,014; filed April 7, 2004); 3) CUFFLESS BLOOD PRESSURE MONITOR AND ACCOMPANYING WEB SERVICES INTERFACE (U.S.S.N. 10/810,237; filed March 26, 2004); 4) VITAL SIGN MONITOR FOR ATHLETIC APPLICATIONS (U.S.S.N; filed September 13, 2004); 5) CUFFLESS BLOOD PRESSURE MONITOR AND ACCOMPANYING WIRELESS MOBILE DEVICE (U.S.S.N. 10/967,511; filed October 18, 2004); 6) BLOOD PRESSURE MONITORING DEVICE FEATURING A CALIBRATION-BASED ANALYSIS (U.S.S.N. 10/967,610; filed October 18, 2004); 7) PERSONAL COMPUTER-BASED VITAL SIGN MONITOR (U.S.S.N. 10/906,342; filed February 15, 2005); 8) PATCH SENSOR FOR MEASURING BLOOD PRESSURE WITHOUT A CUFF (U.S.S.N. 10/906,315; filed February 14, 2005); 9) PATCH SENSOR FOR MEASURING VITAL SIGNS (U.S.S.N. 11/160,957; filed July 18, 2005); 10) WIRELESS, INTERNET-BASED SYSTEM FOR MEASURING VITAL SIGNS FROM A PLURALITY OF PATIENTS IN A HOSPITAL OR MEDICAL CLINIC (U.S.S.N. 11/162,719; filed September 9, 2005); 11) HAND-HELD MONITOR FOR MEASURING VITAL SIGNS (U.S.S.N. 11/162,742; filed September 21, 2005); 12) CHEST STRAP FOR MEASURING VITAL SIGNS (U.S.S.N. 11/306,243; filed December 20, 2005); 13) SYSTEM FOR MEASURING VITAL SIGNS USING AN OPTICAL MODULE FEATURING A GREEN LIGHT SOURCE (U.S.S.N. 11/307,375; filed February 3, 2006); 14) BILATERAL DEVICE, SYSTEM AND METHOD FOR MONITORING VITAL SIGNS (U.S.S.N. 11/420,281; filed May 25, 2006); 15) SYSTEM FOR MEASURING VITAL SIGNS USING BILATERAL PULSE TRANSIT TIME (U.S.S.N. 11/420,652; filed May 26, 2006); 16) BLOOD PRESSURE MONITOR (U.S.S.N. 11/530,076; filed September 8, 2006); 17) TWO-PART PATCH SENSOR FOR MONITORING VITAL SIGNS (U.S.S.N. 11/558,538; filed November 10, 2006); and, 18) MONITOR FOR MEASURING VITAL SIGNS AND RENDERING VIDEO IMAGES (U.S.S.N. 11/682,177; filed March 5, 2007).

Other embodiments are not within the scope of the invention. For example, other measurement techniques, such as conventional oscillometry measured during deflation, can be used to determine SYS for the above-described algorithms. Additionally, processing units and probes for measuring pulse oximetry similar to those described above can be modified and worn on other portions of the patient's body. For example, optical sensors with finger-ring configurations can be worn on fingers other than the thumb. Or they can be modified to attach to other conventional sites for measuring SpO2, such as the ear, forehead, and bridge of the nose. In these embodiments the processing unit can be worn in places other than the wrist, such as around the neck (and supported, e.g., by a lanyard) or on the patient's waist (supported, e.g., by a clip that attaches to the patient's belt). In still other embodiments the probe and processing unit are integrated into a single unit.

In other embodiments, a set of body-worn monitors can continuously monitor a group of patients, wherein each patient in the group wears a body-worn monitor similar to those described herein. Additionally, each body-worn monitor can be augmented with a location sensor. The location sensor includes a wireless component and a location-processing component that receives a signal from the wireless component and processes it to determine a physical location of the patient. A processing component (similar to that described above) determines from the time-dependent waveforms at least one vital sign, one motion parameter, and an alarm parameter calculated from the combination of this information. A wireless transceiver transmits the vital sign, motion parameter, location of the patient, and alarm parameter through a wireless system. A remote computer system featuring a display and an interface to the wireless system receives the information and displays it on a user interface for each patient in the group.

In embodiments, the interface rendered on the display at the central nursing station features a field that displays a map corresponding to an area with multiple sections. Each section corresponds to the location of the patient and includes, e.g., the patient's vital signs, motion parameter, and alarm parameter. For example, the field can display a map corresponding to an area of a hospital (e.g. a hospital bay or emergency room), with each section corresponding to a specific bed, chair, or general location in the area. Typically the display renders graphical icons corresponding to the motion and alarm parameters for each patient in the group. In other embodiments, the body-worn monitor includes a graphical display that renders these parameters directly on the patient.

Typically the location sensor and the wireless transceiver operate on a common wireless system, e.g. a wireless system based on 802.11 (i.e. 'WiFi'), 802.15.4 (i.e. 'Bluetooth'), or cellular (e.g. CDMA, GSM) protocols. In this case a location is determined by processing the wireless signal with one or more algorithms known in the art. These include, for example, triangulating signals received from at least three different base stations, or simply estimating a location based on signal strength and proximity to a particular base station. In still other embodiments the location sensor includes a conventional global positioning system (GPS) that processes signals from orbiting satellites to determine patient's position.

The body-worn monitor can include a first voice interface, and the remote computer can include a second voice interface that integrates with the first voice interface. The location sensor, wireless transceiver, and first and second voice interfaces can all operate on a common wireless system, such as one of the above-described systems based on 802.11 or cellular protocols. The remote computer, for example, can be a monitor that is essentially identical to the monitor worn by the patient, and can be carried or worn by a medical professional. In this case the monitor associated with the medical professional features a GUI wherein the user can select to display information (e.g. vital signs, location, and alarms) corresponding to a particular patient. This monitor can also include a voice interface so the medical professional can communicate directly with the patient.

In other embodiments, the electrodes connect to the ECG circuit using a wireless interface. Here, each electrode includes a small, battery-powered microprocessor, radio, and analog-to-digital converter. The analog-to-digital converter converts an analog signal measured by the electrode into a digital signal. The radio then sends the digital signal to the ECG circuit, which processes signals from multiple electrodes using a differential amplifier to determine both ECG and IP waveforms as described above.

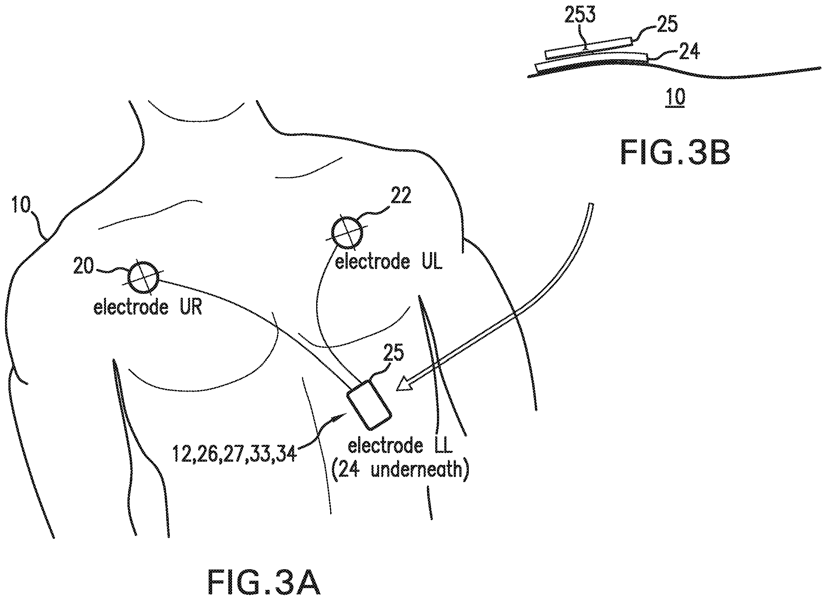

Figs. 3A, 3B show yet another alternate embodiment of the invention wherein a sensor module 25 attaches to the belly of a patient 10 using an electrode 24 normally attached to the lower left-hand portion of the patient's torso. Specifically, the sensor module 25 includes a connector 253 featuring an opening that receives the metal snap or rivet present on most disposable ECG electrodes. Connecting the connector 253 to the electrode's rivet holds the sensor module 25 in place. This configuration reduces the number of cables in the body-worn monitor, and additionally secures an accelerometer 12 to the patient's belly. This is typically the part of their torso that undergoes the greatest motion during respiration, and thus generates ACC waveforms with the highest possible signal-to-noise ratio. Also contained within the sensor module 25 are the ECG circuit 26, the IP circuit 27, microprocessor 33, and a temperature sensor 34.

To measure IP and ECG waveforms, the sensor module 25 connects through cables to electrodes 20, 22 attached, respectively, to the upper right-hand and left-hand portions of the patient's torso. This system measures RR using the peak counting, adaptive filtering, and FFT-based approaches described above, and has the additional advantage of measuring a relatively large ACC signals indicating respiration-induced motions of the patient's belly. As described above, these signals are typically generated by the z-axis of the accelerometer 12, which is normal to the patient's torso. ACC signals along the x and y-axes can be additionally processed to determine the patient's posture and activity level, as described above. Once RR and these motion-related properties are measured, a transceiver in the sensor module (not shown in the figure) transmits them in the form of a digital data stream through a cable to the wrist-worn transceiver for further processing.

Still other embodiments are within the scope of the following claims.

**Claims**

1. A system for measuring respiratory rate from a patient, comprising:

    an impedance pneumography sensor connected to at least two electrodes, the impedance pneumography sensor comprising a processing circuit configured to process signals from the at least two electrodes to measure an impedance pneumography signal from the patient;
    a motion sensor connected to the patient and configured to measure at least one motion signal corresponding to motion of a portion of the patient's body to which it is attached; and
    a processing system configured to:

        determine a motion parameter from the motion signal; and
        based on the motion parameter, select one of the following algorithms to process at least one of the motion and impedance pneumography signals to determine the patient's respiratory rate:

            i) a first algorithm comprising a step for counting breathing-induced pulses in the impedance pneumography signal to determine respiratory rate;
            ii) a second algorithm comprising collectively processing both the motion signal and impedance pneumography signal to determine a digital filter, and then processing one of the motion signal and impedance pneumography signal with the digital filter to determine respiratory rate, or
            iii) a third algorithm comprising processing the impedance pneumography signal to determine a frequency-domain impedance pneumography spectrum, processing the motion signal to determine a frequency-domain motion spectrum, collectively processing both the impedance pneumography spectrum and motion spectrum to remove motion components from the impedance pneumography spectrum and then determining respiratory rate.

2. The system of claim 1,
    wherein the processing system is worn on the patient's body and connected to the impedance pneumography sensor and the motion sensor,
    wherein the processing system is further configured to i) determine motion frequency components from the frequency-domain motion spectrum; ii) remove frequency components from the impedance pneumography spectrum corresponding to the motion frequency components to generate a processed frequency-domain impedance pneumography spectrum; and iii) analyze the processed frequency-domain impedance pneumography spectrum to determine respiratory rate.

**3.** The system of claim 1, further comprising a sensor module that comprises both the impedance pneumography sensor and the motion sensor.

**4.** The system of claim 3, wherein the sensor module

(A) is configured to be worn on the patient's torso,
wherein the sensor module optionally comprises at least one analog-to-digital converter configured to digitize the impedance pneumography signal,
wherein the sensor module optionally comprises at least one analog-to-digital converter configured to digitize the motion signal and

(a) optionally further comprising a serial transceiver configured to transmit digitized versions of the impedance pneumography signal and motion signal to the processing system, wherein the processing system optionally is configured to be worn on the patient's wrist, or
(b) wherein the sensor module optionally comprises the processing system, or
(c) optionally further comprising a wireless transceiver,

wherein the processing system optionally is remote from the patient, and the wireless transceiver is configured to wirelessly transmit digitized versions of the impedance pneumography signal and motion signal to the processing system,
wherein the processing system optionally is further configured to wireless transmit a value describing respiratory rate to a second processor worn on the patient's body,
wherein the second processor optionally is configured to display the value describing respiratory rate on the patient's body;
(B) further comprises a temperature sensor;
(C) further comprises a sensor for measuring an electrocardiogram; or
(D) comprises a housing, the housing comprising a connector that connects directly to an electrode worn on the patient's torso.

**5.** The system of claim 1, wherein the motion sensor is an accelerometer,
wherein the accelerometer is optionally configured to generate a unique motion waveform for each axis of a coordinate system,
wherein the processing system optionally is further configured to process each unique motion waveform to determine a posture corresponding to the patient.

**6.** A method for measuring respiratory rate from a patient, comprising the following steps:

(a) measuring a time-dependent impedance pneumography signal from the patient with an impedance pneumography sensor connected to at least two electrodes, the impedance pneumography signal representing a time-dependent capacitance change in the patient's torso;
(b) measuring at least one time-dependent motion signal with a motion sensor connected to the patient, the at least one motion signal corresponding to motion of a portion of the patient's body to which the motion sensor is attached;
(c) analyzing the motion signal to determine a motion parameter; and
(d) based on the motion parameter, selecting one of the following algorithms to process at least one of the motion and impedance pneumography signals to determine the patient's respiratory rate:

i) a first algorithm comprising a step for counting breathing-induced pulses in the impedance pneumography signal to determine respiratory rate; and
ii) a second algorithm comprising collectively processing both the motion signal and impedance pneumography signal to determine a digital filter, and then processing one of the motion signal and impedance pneumography signal with the digital filter to determine respiratory rate, or
iii) a third algorithm comprising

(a') transforming the time-dependent impedance pneumography signal into a frequency-dependent impedance pneumography spectrum;

(b') transforming the time-dependent motion signal into a frequency-dependent motion spectrum;

(c') determining motion components from the frequency-dependent motion spectrum that correspond to the patient's motion;

(d') removing the motion components, or components calculated therefrom, from the frequency-dependent impedance pneumography spectrum to generate a processed frequency-dependent impedance pneumography spectrum; and

(e') analyzing the processed frequency-dependent impedance pneumography spectrum to determine the patient's respiratory rate.

7.  The method of claim 6, wherein step (e') comprises taking a Fourier Transform of the time-dependent impedance pneumography signal, wherein step (e') optionally comprises calculating a power spectrum of the time-dependent impedance pneumography signal.

8.  The method of claim 6, wherein step (b') comprises calculating a Fourier Transform of the time-dependent motion signal, wherein step (b') optionally comprises calculating a power spectrum of the time-dependent motion signal.

9.  The method of claim 6, wherein step (c') comprises calculating a peak in the frequency-dependent motion spectrum, the peak corresponding to the motion components, wherein

(A) the method optionally further comprises processing the peak in the frequency-dependent motion spectrum to determine a digital filter,

wherein the method optionally further comprises generating an alarm if the patient's respiratory rate is greater than a first pre-determined threshold, or less than a second pre-determined threshold, the method optionally further comprising processing the patient's respiratory rate and the posture corresponding to the patient to generate the alarm; or wherein step (d') optionally further comprises processing the frequency-dependent impedance pneumography spectrum with a smoothing function or with an averaging function after applying the digital filter to generate the processed frequency-dependent impedance pneumography spectrum, or

wherein step (e') optionally further comprises analyzing the processed frequency-dependent impedance pneumography spectrum to determine a spectral peak, the spectral peak corresponding to the patient's respiratory rate,

wherein the method optionally further comprises determining a passband for the digital filter by processing the peak in the frequency-dependent motion spectrum, the passband configured to pass frequency components lying therein, wherein step (d') optionally further comprises processing the frequency-dependent impedance pneumography spectrum with the digital filter to remove motion components and generate the processed frequency-dependent impedance pneumography spectrum; or

(B) wherein step (d') optionally comprises (i) subtracting the motion components from the frequency-dependent impedance pneumography spectrum to generate the processed frequency-dependent impedance pneumography spectrum or (ii) dividing the motion components from the frequency-dependent impedance pneumography spectrum to generate the processed frequency-dependent impedance pneumography spectrum.

10. The method of claim 6, wherein the motion sensor is an accelerometer,

wherein the accelerometer is optionally configured to generate a unique motion waveform for each axis of a coordinate system, the method optionally further comprising processing each unique motion waveform to determine a posture corresponding to the patient.

**Patentansprüche**

1.  System zur Messung der Atemfrequenz bei einem Patienten, umfassend:

einen Impedanz-Pneumographie-Sensor, der an mindestens zwei Elektroden angeschlossen ist, wobei der Impedanz-Pneumographie-Sensor eine Verarbeitungsschaltung aufweist, die zur Verarbeitung von Signalen von den mindestens zwei Elektroden zur Messung eines Impedanz-Pneumographie-Sensors bei dem Patienten konfiguriert ist;

einen Bewegungssensor, der mit dem Patienten verbunden ist und zur Messung mindestens eines Bewegungssignals, das der Bewegung des Körperteils des Patienten entspricht, an dem er befestigt ist, konfiguriert ist; und

ein Verarbeitungssystem, das konfiguriert ist:

zur Bestimmung eines Bewegungsparameters aus dem Bewegungssignal; und
basierend auf dem Bewegungsparameter, zum Auswählen eines der folgenden Algorithmen zur Verarbeitung mindestens eines der Bewegungs- und Impedanz-Pneumographie-Signale zur Bestimmung der Atemfrequenz des Patienten:

i) ein erster Algorithmus, der einen Schritt zum Zählen atmungsinduzierter Impulse im Impedanz-Pneumographie-Signal zur Bestimmung der Atemfrequenz umfasst;
ii) ein zweiter Algorithmus, der zusammengefasstes Verarbeiten sowohl des Bewegungssignals als auch des Impedanz-Pneumographie-Signals zur Bestimmung eines digitalen Filters umfasst, und dann Verarbeiten des Bewegungssignals und des Impedanz-Pneumographie-Signals mit dem digitalen Filter zur Bestimmung der Atemfrequenz umfasst, oder
iii) ein dritter Algorithmus, der Verarbeiten des Impedanz-Pneumographie-Signals zur Bestimmung eines Frequenzbereichs-Impedanz-Pneumographie-Spektrums umfasst, Verarbeiten des Bewegungssignals zur Bestimmung eines Frequenzbereichsbewegungsspektrums, zusammengefastes Verarbeiten sowohl des Impedanz-Pneumographie-Spektrum als auch des Bewegungsspektrums, um Bewegungskomponenten aus dem Impedanz-Pneumographie-Spektrum zu entfernen und dann Bestimmen der Atemfrequenz umfasst.

2. System nach Anspruch 1,
wobei das Verarbeitungssystem am Körper des Patienten getragen wird und an den Impedanz-Pneumographie-Sensor und den Bewegungssensor angeschlossen ist,
wobei das Verarbeitungssystem ferner i) zum Bestimmen von Bewegungsfrequenzkomponenten im Frequenzbereichsbewegungsspektrum; ii) zum Entfernen von Frequenzkomponenten aus dem Impedanz-Pneumographie-Spektrum, die den Bewegungsfrequenzkomponenten entsprechen, um ein verarbeitetes Frequenzbereichs-Impedanz-Pneumographie-Spektrums zu erzeugen und iii) zum Analysieren des verarbeiteten Frequenzbereichs-Impedanz-Pneumographie-Spektrums, um die Atemfrequenz zu bestimmen, konfiguriert ist.

3. System nach Anspruch 1, das weiterhin ein Sensormodul umfasst, das sowohl den Impedanz-Pneumographie-Sensor als auch den Bewegungssensor umfasst.

4. System nach Anspruch 3, wobei das Sensormodul

(A) so konfiguriert ist, dass es am Torso des Patienten getragen wird,
wobei das Sensormodul optional mindestens einen Analog-Digital-Wandler umfasst, der zum Digitalisieren des Impedanz-Pneumographie-Signals konfiguriert ist,
wobei das Sensormodul optional mindestens einen Analog-Digital-Wandler umfasst, der zum Digitalisieren des Bewegungssignals konfiguriert ist, und

(a) optional ferner einen seriellen Sende-Empfänger umfasst, der zum Übertragen digitalisierter Versionen des Impedanz-Pneumographie-Signals und des Bewegungssignals an das Verarbeitungssystem konfiguriert ist, wobei das Verarbeitungssystem optional so konfiguriert ist, dass es am Handgelenk des Patienten getragen wird, oder
(b) wobei das Sensormodul optional das Verarbeitungssystem umfasst, oder
(c) optional ferner einen Funksende-Empfänger umfasst,

wobei sich optional das Verarbeitungssystem vom Patienten entfernt befindet und der Funksende-Empfänger so konfiguriert ist, dass er digitalisierte Versionen des Impedanz-Pneumographie-Signals und des Bewegungssignals drahtlos an das Verarbeitungssystem überträgt,
wobei das Verarbeitungssystem optional ferner zum Übertragen eines Werts per Funk, der die Atemfrequenz beschreibt, an einen zweiten Prozessor, der am Körper des Patienten getragen wird, konfiguriert ist, wobei der zweite Prozessor optional zum Anzeigen des Werts, der die Atemfrequenz beschreibt, am Körper des Patienten konfiguriert ist;
(B) ferner einen Temperatursensor aufweist;
(C) ferner einen Sensor zum Messen eines Elektrokardiogramms aufweist; oder
(D) ein Gehäuse aufweist, wobei das Gehäuse einen Verbinder aufweist, der direkt mit einer am Torso des Patienten getragenen Elektrode verbunden wird.

**5.** System nach Anspruch 1, wobei der Bewegungssensor ein Beschleunigungsmesser ist, wobei der Beschleunigungsmesser optional zur Erzeugung einer eindeutigen Bewegungswellenform für jede Achse eines Koordinatensystems konfiguriert ist,
wobei das Verarbeitungssystem optional ferner zum Verarbeiten jeder eindeutigen Bewegungswellenform zum Bestimmen einer dem Patienten entsprechenden Haltung konfiguriert ist.

**6.** Verfahren zum Messen einer Atemfrequenz bei einem Patienten, umfassend:

(a) Messen eines zeitabhängigen Impedanz-Pneumographie-Signals bei dem Patienten mit einem Impedanz-Pneumographie-Sensor, der mit mindestens zwei Elektroden verbunden ist, wobei das Impedanz-Pneumographie-Signal eine zeitabhängige Kapazitätsänderung im Torso des Patienten darstellt;
(b) Messen mindestens eines zeitabhängigen Bewegungssignals mit einem an den Patienten angeschlossenen Bewegungssensor, wobei das mindestens eine Bewegungssignal der Bewegung des Körperteils des Patienten entspricht, an dem der Bewegungssensor befestigt ist;
(c) Analysieren des Bewegungssignals zum Bestimmen eines Bewegungsparameters; und
(d) basierend auf dem Bewegungsparameter, Auswählen eines der folgenden Algorithmen zum Verarbeiten mindestens eines der Bewegungs- und Impedanz-Pneumographie-Signale zur Bestimmung der Atemfrequenz des Patienten:

i) ein erster Algorithmus, der einen Schritt zum Zählen atmungsinduzierter Impulse im Impedanz-Pneumographie-Signal zum Bestimmen der Atemfrequenz umfasst; und
ii) ein zweiter Algorithmus, der zusammengefasstes Verarbeiten sowohl des Bewegungssignals als auch des Impedanz-Pneumographie-Signals zum Bestimmen eines digitalen Filters und dann Verarbeiten des Bewegungssignals und des Impedanz-Pneumographie-Signals mit dem digitalen Filter zum Bestimmen der Atemfrequenz umfasst, oder
iii) ein dritter Algorithmus, der umfasst

(a') Umwandeln des zeitabhängigen Impedanz-Pneumographie-Signals in ein frequenzabhängiges Impedanz-Pneumographie-Spektrum;
(b') Umwandeln des zeitabhängigen Bewegungssignals in ein frequenzabhängiges Bewegungsspektrum;
(c') Bestimmen von Bewegungskomponenten aus dem frequenzabhängigen Bewegungsspektrum, die der Bewegung des Patienten entsprechen;
(d') Entfernen der Bewegungskomponenten oder der daraus berechneten Komponenten aus dem frequenzabhängigen Impedanz-Pneumographie-Spektrum, um ein verarbeitetes frequenzabhängiges Impedanz-Pneumographie-Spektrums zu erzeugen; und
(e') Analysieren des verarbeiteten frequenzabhängigen Impedanz-Pneumographie-Spektrums zum Bestimmen der Atemfrequenz des Patienten.

**7.** Verfahren nach Anspruch 6, wobei Schritt (e') eine Fourier-Transformation des zeitabhängigen Impedanz-Pneumographie-Signals umfasst, wobei der Schritt (e') optional die Berechnung eines Leistungsspektrums des zeitabhängigen Impedanz-Pneumographie-Signals umfasst.

**8.** Verfahren nach Anspruch 6, wobei der Schritt (b') die Berechnung einer Fourier-Transformation des zeitabhängigen Bewegungssignals umfasst, wobei der Schritt (b') optional die Berechnung eines Leistungsspektrums des zeitabhängigen Bewegungssignals umfasst.

**9.** Verfahren nach Anspruch 6, wobei der Schritt (c') Berechnen eines Spitzenwerts im frequenzabhängigen Bewegungsspektrum umfasst, wobei der Spitzenwert den Bewegungskomponenten entspricht, wobei

(A) das Verfahren optional ferner Verarbeiten des Spitzenwerts im frequenzabhängigen Bewegungsspektrum zum Bestimmen eines digitalen Filters umfasst,
wobei das Verfahren optional ferner das Erzeugen eines Alarms umfasst, wenn die Atemfrequenz des Patienten höher als ein erster vorbestimmter Schwellenwert oder niedriger als ein zweiter vorbestimmter Schwellenwert ist, wobei das Verfahren optional ferner Verarbeiten der Atemfrequenz des Patienten und der dem Patienten entsprechenden Haltung zum Erzeugen des Alarms umfasst; oder
wobei der Schritt (d') optional ferner Verarbeiten des frequenzabhängigen Impedanz-Pneumographie-Spektrums mit einer Glättungsfunktion oder mit einer Mittelungsfunktion nach dem Anlegen des digitalen Filters zur Erzeugung des verarbeiteten frequenzabhängigen Impedanz-Pneumographie-Spektrums umfasst, oder wobei der Schritt (e') optional ferner Analysieren des verarbeiteten frequenzabhängigen Impedanz-Pneumographie-Spektrums zum Bestimmen eines spektralen Spitzenwerts umfasst, wobei der spektrale Spitzenwert der Atemfrequenz des Patienten entspricht, wobei das Verfahren weiterhin Bestimmen eines Durchlassbands für den digitalen Filter durch Verarbeiten des Spitzenwerts im frequenzabhängigen Bewegungsspektrum umfasst, wobei das Durchlassband so konfiguriert ist, dass darin liegende Frequenzkomponenten passieren gelassen werden, wobei der Schritt (d') optional ferner Verarbeiten des frequenzabhängigen Impedanz-Pneumographie-Spektrums mit dem digitalen Filter umfasst, um Bewegungskomponenten zu entfernen und ein verarbeitetes frequenzabhängiges Impedanz-Pneumographie-Spektrums zu erzeugen; oder

(B) wobei Schritt (d') optional (i) Subtrahieren der Bewegungskomponenten aus dem frequenzabhängigen Impedanz-Pneumographie-Spektrum zum Erzeugen des verarbeiteten frequenzabhängigen Impedanz-Pneumographie-Spektrums, oder (ii) Trennen der Bewegungskomponenten vom frequenzabhängigen Impedanz-Pneumographie-Spektrum zum Erzeugen des verarbeiteten frequenzabhängigen Impedanz-Pneumographie-Spektrums umfasst.

10. Verfahren nach Anspruch 6, wobei der Bewegungssensor ein Beschleunigungsmesser ist, wobei der Beschleunigungsmesser optional zum Erzeugen einer eindeutigen Bewegungswellenform für jede Achse eines Koordinatensystems konfiguriert ist, wobei das Verfahren optional ferner Verarbeiten jeder eindeutigen Bewegungswellenform zur Bestimmung der Haltung des Patienten umfasst.

**Revendications**

1. Système de mesure de la fréquence respiratoire d'un patient, comprenant :

un capteur de pneumographie d'impédance connecté à au moins deux électrodes, le capteur de pneumographie d'impédance comprenant un circuit de traitement conçu pour traiter des signaux en provenance desdites électrodes pour mesurer le signal de pneumographie d'impédance provenant du patient ;
un capteur de mouvement connecté au patient et conçu pour mesurer au moins un signal de mouvement correspondant au mouvement d'une partie du corps du patient à laquelle il est fixé ; et
un système de traitement conçu pour :

déterminer un paramètre de mouvement du signal de mouvement ; et
en fonction du paramètre de mouvement, sélectionner l'un des algorithmes suivants pour traiter au moins l'un des signaux de pneumographie d'impédance et de mouvement pour déterminer la fréquence respiratoire du patient :

i) un premier algorithme comprenant une étape servant à compter les impulsions induites par la respiration dans le signal de pneumographie d'impédance pour déterminer la fréquence respiratoire ;
ii) un deuxième algorithme consistant à traiter ensemble aussi bien le signal de mouvement que le signal de pneumographie d'impédance pour déterminer un filtre numérique et ensuite traiter l'un parmi le signal de mouvement et le signal de pneumographie d'impédance à l'aide du filtre numérique pour déterminer la fréquence respiratoire, ou
iii) un troisième algorithme consistant à traiter le signal de pneumographie d'impédance pour déterminer le spectre de pneumographie d'impédance en domaine fréquentiel, traiter le signal de mouvement pour déterminer un spectre de mouvement en domaine fréquentiel, traiter ensemble aussi bien le spectre de pneumographie d'impédance et le spectre de mouvement pour éliminer les composantes de mouvement du spectre de pneumographie d'impédance et ensuite pour déterminer la fréquence respiratoire.

2. Système selon la revendication 1,
dans lequel le système de traitement est placé sur le corps du patient et connecté au capteur de pneumographie d'impédance et au capteur de mouvement,
dans lequel le système de traitement est en outre conçu pour i) déterminer les composantes de fréquence de mouvement à partir du spectre de mouvement en domaine fréquentiel ; ii) éliminer les composantes de fréquence du spectre de pneumographie d'impédance correspondant aux composantes de fréquence de mouvement pour générer un spectre de pneumographie d'impédance en domaine fréquentiel traité ; et iii) analyser le spectre de pneumographie d'impédance en domaine fréquentiel traité pour déterminer la fréquence respiratoire.

**3.** Système selon la revendication 1, comprenant en outre un module de capteur qui comprend aussi bien le capteur de pneumographie d'impédance que le capteur de mouvement.

**4.** Système selon la revendication 3, dans lequel le module de capteur

(A) est conçu pour être placé sur le torse du patient,
dans lequel le module de capteur comprend éventuellement au moins un convertisseur analogique-numérique conçu pour numériser le signal de pneumographie d'impédance,
dans lequel le module de capteur comprend éventuellement au moins un convertisseur analogique-numérique conçu pour numériser le signal de mouvement et

(a) comprenant en outre éventuellement un émetteur-récepteur en série conçu pour transmettre des versions numériques du signal de pneumographie d'impédance et du signal de mouvement au système de traitement, dans lequel le système de traitement est conçu éventuellement pour être porté au poignée par le patient, ou
(b) dans lequel le module de capteur comprend éventuellement le système de traitement, ou
(c) comprenant en outre éventuellement un émetteur-récepteur sans fil,

dans lequel le système de traitement est éventuellement à distance du patient et l'émetteur-récepteur sans fil est conçu pour transmettre par le réseau sans fil des versions numérisées du signal de pneumographie d'impédance et du signal de mouvement au système de traitement,
dans lequel le système de traitement en option est en outre conçu éventuellement pour transmettre par le réseau sans fil une valeur décrivant la fréquence respiratoire à un second processeur placé sur le corps du patient,
dans lequel le second processeur est conçu éventuellement pour afficher la valeur décrivant la fréquence respiratoire sur le corps du patient ;
(B) comprend en outre un capteur de température ;
(C) comprend en outre un capteur de mesure d'un électrocardiogramme ; ou
(D) comprend un boîtier, le boîtier comprenant un connecteur qui se connecte directement à une électrode placée sur le torse du patient.

**5.** Système selon la revendication 1, dans lequel le capteur de mouvement est un accéléromètre,
dans lequel l'accéléromètre est conçu éventuellement pour générer une forme d'onde de mouvement unique pour chaque axe d'un système de coordonnées,
dans lequel le système de traitement est conçu éventuellement pour traiter chaque forme d'onde de mouvement unique pour déterminer une posture correspondant au patient.

**6.** Procédé de mesure de la fréquence respiratoire provenant d'un patient, comprenant les étapes consistant à :

(a) mesurer un signal de pneumographie d'impédance dépendant du temps provenant du patient à l'aide d'un capteur de pneumographie d'impédance connecté à au moins deux électrodes, le signal de pneumographie d'impédance représentant une modification de capacité électrique dépendante du temps dans le torse du patient ;
(b) mesurer au moins un signal de mouvement dépendant du temps à l'aide d'un capteur de mouvement connecté au patient, l'au moins un signal de mouvement correspondant au mouvement d'une partie du corps du patient à laquelle est fixé le capteur de mouvement ;
(c) analyser le signal de mouvement pour déterminer un paramètre de mouvement ; et
(d) en fonction du paramètre de mouvement, sélecter l'un des algorithmes suivants pour traiter au moins l'un des signaux de pneumographie d'impédance et de mouvement pour déterminer la fréquence respiratoire du patient :

i) un premier algorithme comprenant une étape servant à compter les impulsions induites par la respiration dans le signal de pneumographie d'impédance pour déterminer la fréquence respiratoire ; et
ii) un deuxième algorithme consistant à traiter ensemble aussi bien le signal de mouvement que le signal de pneumographie d'impédance pour déterminer un filtre numérique et ensuite traiter l'un parmi le signal de mouvement et le signal de pneumographie d'impédance à l'aide du filtre numérique pour déterminer la fréquence respiratoire, ou
iii) un troisième algorithme consistant à (a') transformer le signal de pneumographie d'impédance dépendant du temps en un spectre de pneumographie d'impédance dépendant de la fréquence ;

(b') transformer le signal de mouvement dépendant du temps en un spectre de mouvement dépendant de la fréquence ;
(c') déterminer des composantes de mouvement provenant du spectre de mouvement dépendant de la fréquence qui correspondent au mouvement du patient ;
(d') éliminer les composantes de mouvement, ou les composantes calculées partir de celui-ci, du spectre de pneumographie d'impédance dépendant de la fréquence pour générer un spectre de pneumographie d'impédance traité dépendant de la fréquence ; et
(e') analyser le spectre de pneumographie d'impédance traité dépendant de la fréquence pour déterminer la fréquence respiratoire du patient.

**7.** Procédé selon la revendication 6, l'étape (e') consistant à utiliser une transformée de Fourier (e') du signal de pneumographie d'impédance dépendant du temps, l'étape (e') consistant éventuellement à calculer un spectre de puissance du signal de pneumographie d'impédance dépendant du temps.

**8.** Procédé selon la revendication 6, l'étape (b') consistant à calculer une transformée de Fourier du signal de mouvement dépendant du temps, l'étape (b') consistant éventuellement à calculer un spectre de puissance du signal de mouvement du temps.

**9.** Procédé selon la revendication 6, dépendant l'étape (c') consistant à calculer une crête dans le spectre de mouvement en fonction de la fréquence, la crête correspondant aux composantes de mouvement,

(A) le procédé consistant en outre éventuellement à traiter la crête dans le spectre de mouvement dépendant de la fréquence pour déterminer un filtre numérique,
le procédé consistant en outre éventuellement à générer une alarme si la fréquence respiratoire du patient est supérieure à un premier seuil prédéfini, ou inférieure à un second seuil prédéfini, le procédé consistant en outre éventuellement à traiter la fréquence respiratoire du patient et la posture correspondant au patient pour générer l'alarme ; ou
l'étape (d') consistant en outre éventuellement à traiter le spectre de pneumographie d'impédance dépendant de la fréquence au moyen d'une fonction de lissage ou au moyen d'une fonction de moyennage après l'application du filtre numérique pour générer le spectre de pneumographie d'impédance traité dépendant de la fréquence, ou l'étape (e') consistant en outre éventuellement à analyser le spectre de pneumographie d'impédance dépendant de la fréquence pour déterminer une crête spectrale, la crête spectrale correspondant à la fréquence respiratoire du patient,
le procédé consistant en outre éventuellement à déterminer une bande passante pour le filtre numérique par traitement de la crête dans le spectre de mouvement dépendant de la fréquence, la bande passante configurée pour faire passer des composantes de fréquence se trouvant en son sein, l'étape (d') consistant en outre éventuellement à traiter le spectre de pneumographie d'impédance dépendant de la fréquence à l'aide du filtre numérique pour éliminer les composantes de mouvement et générer le spectre de pneumographie d'impédance dépendant de la fréquence ; ou
(B) l'étape (d') consistant éventuellement (i) à soustraire les composantes de mouvement du spectre de pneumographie d'impédance dépendant de la fréquence pour générer le spectre de pneumographie d'impédance traité dépendant de la fréquence ou (ii) diviser les composantes de mouvement du spectre de pneumographie d'impédance dépendant de la fréquence pour générer le spectre de pneumographie d'impédance traité dépendant de la fréquence.

**10.** Procédé selon la revendication 6, dans lequel le capteur de mouvement est un accéléromètre,
dans lequel l'accéléromètre est éventuellement conçu pour générer une forme d'onde de mouvement unique pour chaque axe d'un système de coordonnées, le procédé consistant en outre éventuellement à traiter chaque forme d'onde de mouvement unique pour déterminer une posture correspondant au patient.

FIG.1

FIG.2

253
25
24
10

FIG.3B

10

22

20

electrode UL

electrode UR

25

12,26,27,33,34

electrode LL
(24 underneath)

FIG.3A

EP 2 560 550 B1

Configuration for Indexing cNIBP Measurement (first ~60 seconds)

Configuration for cNIBP/RR Measurements (remaining 3 hours, 59 minutes)

FIG.4A

FIG.4B

FIG.5

FIG.6

EP 2 560 550 B1

170

172

**Remote Server**

store RR values

**Wrist—Worn Transceiver**

collect ACC/IP waveforms
process ACC waveforms to determine no motion is present
process IP waveforms to determine adequate signal quality exists
process IP waveforms with *Algorithm 1* to determine RR values

RR Values

FIG.7

EP 2 560 550 B1

179

count zero
−point crossings
In derivatized
waveform

194

take derivative of
filtered waveform

192

No Measurement

F

SD peaks<$\beta$?
$\varepsilon1>\omega>\varepsilon2$?

196

T

RR

filter IP waveforms
w/12 Hz bandpass filter
SD peaks<$\beta$?
$\varepsilon1>\omega>\varepsilon2$?

190

F

T

RR

188

count zero−point crossings
In derivatized waveform

186

take derivative of
filtered waveform and square value

184

filter IP waveforms
w/ 2.5 Hz bandpass filter

182

determine patient
in supine and not moving

180

FIG.8

★ IDEAL FILTER FOR LOW-FREQUENCY BREATHING

FIG.9

EP 2 560 550 B1

FIG. 10

★ IDEAL FILTER FOR HIGH-FREQUENCY BREATHING

202

**Wrist—Worn Transceiver**

collect ACC/IP waveforms
process ACC waveforms to determine
motion is present
process ACC/IP waveforms with
Algorithm 2 to determine RR values

RR Values

200

**Remote Server**

store RR values

FIG.11

**FIG.12**

219

No Measurement SD peaks<$\beta$?
$\varepsilon1>\omega>\varepsilon2$? RR
F T
232

count zero-point crossings
in derivatized waveform
230

take derivative of
filtered waveform and square value
228

adaptively filter IP waveform
using coefficients
226

determine adaptive filtering
coefficients (on transceiver/server)
224

measure IP/ACC
waveforms
222

determine patient
is moving but not walking
220

FIG.13

No Measurement

SD peaks<$\beta$?
$\varepsilon 1 > \omega > \varepsilon 2$?

RR

F

T

count zero-point crossings
in derivatized waveform

remove motion artifact from
IP signal: $y_R(i) = y(i) - \hat{\theta}\phi(i)$
264

take derivative of
filtered waveform

formulate coefficients of FIR/ARMA
models as column vector: $\hat{\theta} = R_n^{-1}B$
262

219

define column vector B
from $\Sigma$ of $y(i)$ and $\phi(i)$
260

adaptively filter IP waveform
using coefficients

construct a square matrix $R_n$
using $\phi(i)$ values for each data point
258

determine adaptive filtering
coefficients (on transceiver/server)

formulate a column vector $\phi(i)$
for each time-sampled data point
256

measure IP/ACC
waveforms

determine filter type
(ARMA/FIR) and order
254

249

determine patient is
moving but not walking

determine zero mean input/output
signals for $u(i)$, $y(i)$
252

$u(i) \rightarrow$ motion magnitude
$y(i) \rightarrow$ RR signal + motion-induced noise
250

FIG.14

ACC – Bandpass Filtered at 0.01 → 2 Hz

FIG.15A

IP – Bandpass Filtered at 0.01 → 12 Hz

FIG.15B

IP – Adaptive Bandpass Filtered at 0.01 → 1.5 x IP Frequency

FIG.15C

EP 2 560 550 B1

d[IP]/dt (IP*)

FIG.15D

Final Waveform with Breathing Counts

◆ algorithm-detected peak

FIG.15E

ACC initial filter — 290

IP initial filter — 291

IP adaptive filter — 292

IP* zero-point crossing — 293

IP* auto pulse counting — 294

RR
5 breaths/min.

FIG.15F

EP 2 560 550 B1

46

ACC – Bandpass Filtered at 0.01 → 2 Hz

**FIG.16A**

IP – Bandpass Filtered at 0.01 → 12 Hz

**FIG.16B**

IP – Adaptive Bandpass Filtered at 0.01 → 1.5 x IP Frequency

**FIG.16C**

EP 2 560 550 B1

$d[IP]/dt$ (IP*)

**FIG.16D**

Final Waveform with Breathing Counts

◆ algorithm-detected peak

**FIG.16E**

ACC
initial
filter — 290

IP
initial
filter — 291

IP
adaptive
filter — 292

IP*
zero-point
crossing — 293

IP*
auto pulse
counting — 294

RR
*17 breaths/min.*

**FIG.16F**

EP 2 560 550 B1

ACC — Bandpass Filtered at 0.01 → 2 Hz

FIG.17A

IP — Bandpass Filtered at 0.01 → 12 Hz

FIG.17B

IP — Adaptive Bandpass Filtered at 0.01 → 1.5 x IP Frequency

FIG.17C

d[IP]/dt (IP*)

**FIG.17D**

Final Waveform with Breathing Counts

◆ algorithm-detected peak

**FIG.17E**

RR
*38 breaths/min.*

**FIG.17F**

EP 2 560 550 B1

300

302

**Remote Server**

process ACC/IP waveforms with
*Algorithm 4* to determine RR values

store RR values

ACC/IP Waveforms

RR Values

**Wrist–Worn
Transceiver**

collect ACC/IP waveforms
process ACC waveforms to determine
motion is present

store and display RR values

FIG.18

No Measurement FFT power>α? RR
ε1>ω>ε2? (Alarming on Page X)
F T

332

find peak in processed
IP waveform

330

process filtered IP waveform
w/ 4Hz smoothing function

328

remove walking frequency (+/− 0.25 Hz)
from FFT of IP waveform

326

process ACC FFT
to determine walking frequency

324

determine real, imaginary FFTs of
IP/ACC waveforms (on server)

322

determine patient
is walking

320

319

FIG.19

EP 2 560 550 B1

Time-Domain Waveforms

IP — Time Domain

FIG.20A

Time-Domain Waveforms

ACC — Time Domain

FIG.20B

Frequency-Domain Waveforms

IP — Frequency Domain

FIG.20C

Frequency-Domain Waveforms

ACC - Frequency Domain

**FIG.20D**

Frequency-Domain Waveforms

IP - Frequency Domain w/ noise removed using notch filter

**FIG.20E**

Frequency-Domain Waveforms

IP - Frequency Domain processed w/ smoothing filter (4 Hz Low Pass)

**FIG.20F**

Time-Domain Waveforms

IP – Time Domain

FIG.21A

Time-Domain Waveforms

ACC – Time Domain

FIG.21B

Frequency-Domain Waveforms

IP – Frequency Domain

FIG.21C

Frequency-Domain Waveforms

ACC - Frequency Domain

0.5 Hz

380

Frequency (Hz)

# FIG.21D

Frequency-Domain Waveforms

IP - Frequency Domain w/ noise removed using notch filter

382

Frequency (Hz)

# FIG.21E

Frequency-Domain Waveforms

IP - Frequency Domain processed w/ smoothing filter (4 Hz Low Pass)

true respiration rate: 18 breaths/min.

Frequency (Hz)

# FIG.21F

Normal Breathing – Laying Down on Back

IP

Normalized IP Sig. (arb.)

Time (seconds)

FIG.22A

Normal Breathing – Laying Down on Back

ACC

Normalized ACC Sig. (arb.)

Time (seconds)

FIG.22B

FIG.22C

Fast, Shallow Breathing – Laying Down on Back

FIG.23A

Fast, Shallow Breathing – Laying Down on Back

FIG.23B

Fast, Shallow Breathing – Laying Down on Back

actual RR determined by
et-CO2 (48–50 breaths/min.)

FIG.23C

EP 2 560 550 B1

Normal Breathing — Laying Face Down

Time (seconds)

FIG.24A

Normalized IP Sig. (arb.)

IP

Normal Breathing — Laying Face Down

Time (seconds)

FIG.24B

Normalized ACC Sig. (arb.)

ACC

Normal Breathing — Laying Face Down

FFT IP/ACC

ACC-FFT
IP-FFT

actual RR determined by
et-CO2 (7-9 breaths/min.)

FIG.24C

EP 2 560 550 B1

FIG.25A

FIG.25B

FIG.25C

FIG.25D

FIG.25E

No Running Average

FIG.26A

10-pt Running Average

FIG.26B

20-pt Running Average

FIG.26C

50-pt Running Average

FIG.26D

100-pt Running Average

FIG.26E

FIG.27A

FIG.27B

FIG.27C

FIG.27D

FIG.28A

FIG.28B

FIG.28C

FIG.28D

IP Signal (arb.)

Time (seconds)

○1000-pt FFT (40 seconds)

Normalized Signal (arb.)

Frequency (Hz)

⊘500-pt FFT (20 seconds)

Normalized Signal (arb.)

Frequency (Hz)

●250-pt FFT (10 seconds)

Normalized Signal (arb.)

Frequency (Hz)

FFT

FIG.29

FIG.30A

FIG.30B

EP 2 560 550 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0875199 A
- US 12469182 B
- US 8419649 B2
- US 8808188 B2
- US 12138194 B
- US 12650354 B
- US 12559419 B
- US 12171886 B
- US 10709015 B
- US 10709014 B
- US 10810237 B
- US 10967511 B
- US 10967610 B
- US 10906342 B
- US 10906315 B
- US 11160957 B
- US 11162719 B
- US 11162742 B
- US 11306243 B
- US 11307375 B
- US 11420281 B
- US 11420652 B
- US 11530076 B
- US 11558538 B
- US 11682177 B

### Non-patent literature cited in the description

- **FIESELMANN et al.** RR predicts cardiopulmonary arrest for internal medicine patients. *J Gen Intern Med,* 1993, vol. 8, 354-360
- **SUBBE et al.** Effect of introducing the Modified Early Warning score on clinical outcomes, cardio-pulmonary arrests and intensive care utilization in acute medical admissions. *Anaesthesia,* 2003, vol. 58, 797-802
- **GOLDHILL et al.** A physiologically-based early warning score for ward patients: the association between score and outcome. *Anaesthesia,* 2005, vol. 60, 547-553
- **CRETIKOS et al.** The Objective Medical Emergency Team Activation Criteria: a case-control study. *Resuscitation,* 2007, vol. 73, 62-72